(19) Europäisches Patentamt
European Patent Office
Office européen des brevets

(11) EP 3 919 128 A1

(12) **EUROPEAN PATENT APPLICATION**
published in accordance with Art. 153(4) EPC

(43) Date of publication:
**08.12.2021 Bulletin 2021/49**

(21) Application number: **20748429.6**

(22) Date of filing: **28.01.2020**

(51) Int Cl.:
***A61P 43/00*** *(2006.01)*
***C07K 14/475*** *(2006.01)*
***C07K 17/10*** *(2006.01)*
***C07K 19/00*** *(2006.01)*
***A61K 47/60*** *(2017.01)*
***A61K 47/65*** *(2017.01)*
***A61K 38/18*** *(2006.01)*

(86) International application number:
**PCT/JP2020/002864**

(87) International publication number:
**WO 2020/158691 (06.08.2020 Gazette 2020/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **28.01.2019 JP 2019011808**

(71) Applicant: **Toray Industries, Inc.**
**Tokyo, 103-8666 (JP)**

(72) Inventors:
- **SERIZAWA, Takashi**
  **Kamakura-shi, Kanagawa 248-8555 (JP)**
- **MORI, Katsuyuki**
  **Kamakura-shi, Kanagawa 248-8555 (JP)**
- **ASANO, Tomomi**
  **Kamakura-shi, Kanagawa 248-8555 (JP)**
- **SATO, Mikiya**
  **Kamakura-shi, Kanagawa 248-8555 (JP)**

(74) Representative: **Kador & Partner PartG mbB**
**Corneliusstraße 15**
**80469 München (DE)**

# (54) POLYETHYLENE GLYCOL MODIFIED BODY FOR HEPATOCYTE GROWTH FACTOR OR ACTIVE FRAGMENT THEREOF

(57) An objective of the present invention is to provide a variant of a hepatocyte growth factor or an active fragment thereof which can reduce the liver tropism and selectively exert bioactivity in a target tissue in disease other than a liver tissue. The present invention provides a polyethylene glycol-modified form of a hepatocyte growth factor or an active fragment thereof, wherein polyethylene glycol(s) is/are bound to (a) terminus(es) of the hepatocyte growth factor or the active fragment thereof via (a) protease-sensitive peptide(s).

EP 3 919 128 A1

## Description

Technical Field

The present invention relates to a polyethylene glycol-modified form of a hepatocyte growth factor or an active fragment thereof.

Background Art

Hepatocyte growth factor is a growth factor having diverse pharmacological effects and are known to have an anti-apoptotic effect, an angiogenic effect, a vasodilatory effect, an anti-organ fibrosis effect, an anti-epithelial mesenchymal transition effect, and the like, in addition to an originally found hepatocyte proliferative effect. Currently, its clinical applications to various diseases have been attempted.

However, it has been suggested that the hepatocyte growth factor needs to be frequently administered in large amounts for sustaining their pharmacological effects, because the hepatocyte growth factor has an *in vivo* half-life as short as approximately 30 minutes (Non Patent Literature 1). Meanwhile, it has been reported that the administration of the hepatocyte growth factor in large amounts induces an enlargement of the liver ascribable to the hepatocyte proliferation as an adverse effect thereof, because the hepatocyte growth factor has a liver tropism (Non Patent Literature 2).

NK1 composed of N domain and kringle 1 in Non Patent Literature 3, and NK2 composed of N domain, kringle 1 and kringle 2 in Non Patent Literature 4 have been reported as active fragments which are natural splicing variants of the hepatocyte growth factor. Also, NK4 composed of N domain, kringle 1, kringle 2, kringle 3 and kringle 4 has been reported as an active fragment developed by a recombination technique in Non Patent Literature 5. It has been reported that these active fragments have an agonistic activity or an antagonistic activity against c-Met, a receptor tyrosine kinase for hepatocyte growth factor receptor, *in vitro* and *in vivo.* It has also been reported that NK1 has a liver tropism and induces an enlargement of the liver *in vivo* (Non Patent Literatures 3 and 6).

Polyethylene glycol-modified forms aimed mainly at an *in vivo* half-life extending effect have been reported as to the hepatocyte growth factors and NK4, one of the active fragments thereof (Patent Literatures 1 and 2). Polyethylene glycol is a highly biocompatible polymer and is widely used as a modifying agent aimed at *in vivo* half-life extension or immunogenicity reduction of proteins.

In the case of chemically modifying a protein with the polyethylene glycol, it is known that the bioactivity of the protein is decreased or lost, depending on the position of the modification. Patent Literature 1 has reported that, for example, a modified form in which a hepatocyte growth factor is chemically modified directly with a plurality of polyethylene glycol molecules at random can achieve an *in vivo* half-life extending effect on the hepatocyte growth factor, whereas its bioactivity is lowered by 30% or more.

Non Patent Literature 7 has reported a prodrug of *Pseudomonas aeruginosa-derived* exotoxin A chemically modified with polyethylene glycol via a protease-sensitive peptide for rhinovirus-derived 3C protease for the purpose of empirically controlling drug-derived toxicity.

Citation List

Patent Literature

Patent Literature 1: International Publication No. WO 1996/28475
Patent Literature 2: JP Patent Publication (Kokai) No. 2010-174034 A (2010)

Non Patent Literature

Non Patent Literature 1: Liu K.X. et al., The American Journal of Physiology, 1998, Vol. 275, p. E835-E842
Non Patent Literature 2: Sakata H. et al., Cell Growth & Differentiation, 1996, Vol. 7, p. 1513-1523
Non Patent Literature 3: Jakubczak J.L. et al., Molecular and Cellular Biology, 1998, Vol. 18, No. 3, p. 1275-1283
Non Patent Literature 4: Otsuka T. et al., Molecular and Cellular Biology, 2000, Vol. 20, No. 6, p. 2055-2065
Non Patent Literature 5: Date K. et al., FEBS Letters, 1997, Vol. 420, No. 1, p. 1-6
Non Patent Literature 6: Ross J. et al., Gastroenterology, 2012, Vol. 142, p. 897-906
Non Patent Literature 7: Stefan N. et al., Bioconjugate Chemistry, 2014, Vol. 25, p. 2144-2156

## Summary of Invention

### Technical Problem

In Non Patent Literature 7, however, *in vivo* and *ex vivo* functional verification has not been carried out on a prodrug of exotoxin A which is a toxin protein derived from *Pseudomonas aeruginosa.* Furthermore, the prodrug of exotoxin A disclosed in Non Patent Literature 7 has undergone chemical modification with polyethylene glycol via a protease-sensitive peptide at two locations, i.e., at an end of exotoxin A and in the middle of its sequence, in order to suppress the bioactivity of the exotoxin A. In general, peptide addition or chemical modification with polyethylene glycol in the middle of the sequence of a protein entails uncertainty from the viewpoint of the maintenance of bioactivity originally possessed by the protein, because of a high level of difficulty in production and because an unnecessary amino acid sequence derived from the protease-sensitive peptide remains in the middle of the structure of the protein even after cleavage by the protease. Thus, the technique described in Non Patent Literature 7 has poor generality and seems to be not easily applicable to other proteins.
Non Patent Literature 7 neither discloses nor suggests a prodrug of a hepatocyte growth factor or an active fragment thereof and neither discloses nor suggests *in vivo* protease that may be exploited for the design of the prodrug for the purpose of reducing liver tropism of the hepatocyte growth factor or the active fragment thereof.
Hence, it has been desired to develop a variant of a hepatocyte growth factor or an active fragment thereof which can reduce liver tropism of the hepatocyte growth factor or the active fragment thereof and selectively exert the bioactivity of the hepatocyte growth factor or the active fragment thereof in a target disease tissue other than a liver tissue.
Accordingly, an objective of the present invention is to provide a variant of a hepatocyte growth factor or an active fragment thereof which can solve the problems described above.

### Solution to Problem

The present inventors have conducted diligent studies to attain the objective and consequently completed the present invention by finding that a hepatocyte growth factor or an active fragment thereof is chemically modified with the polyethylene glycol(s) via (a) protease-sensitive peptide(s), whereby liver tropism of the hepatocyte growth factor or the active fragment thereof can be reduced while the bioactivity of the hepatocyte growth factor or the active fragment thereof can be selectively exerted by the cleavage of the protease-sensitive peptide in a target disease tissue.
Specifically, the present invention encompasses the following (1) to (7).

(1) A polyethylene glycol-modified form of a hepatocyte growth factor or an active fragment thereof, wherein the polyethylene glycol(s) is/are bound to any end of the hepatocyte growth factor or the active fragment thereof via (a) protease-sensitive peptide(s).
(2) The polyethylene glycol-modified form of a hepatocyte growth factor or an active fragment thereof according to (1), wherein the protease-sensitive peptide(s) is/are (an) ADAM17-sensitive peptide(s) or (a) thrombin-sensitive peptide(s).
(3) The polyethylene glycol-modified form of a hepatocyte growth factor or an active fragment thereof according to (1), wherein the protease-sensitive peptide(s) has/have an amino acid sequence represented by any one of SEQ ID NOs: 8 to 20 in the sequence listing.
(4) The polyethylene glycol-modified form of a hepatocyte growth factor or an active fragment thereof according to any of (1) to (3), wherein (a) number-average molecular weight(s) of the polyethylene glycol(s) is/are 20000 to 100000.
(5) The polyethylene glycol-modified form of a hepatocyte growth factor or an active fragment thereof according to any of (1) to (4), wherein the active fragment of the hepatocyte growth factor is NK1.
(6) The polyethylene glycol-modified form of a hepatocyte growth factor or an active fragment thereof according to any of (1) to (5), wherein the active fragment of the hepatocyte growth factor has the amino acid sequence represented by SEQ ID NO: 2 in the sequence listing.
(7) A medicament comprising a polyethylene glycol-modified form of a hepatocyte growth factor or an active fragment thereof according to any of (1) to (6) as an active ingredient.

### Advantageous Effects of Invention

The polyethylene glycol-modified form of a hepatocyte growth factor or an active fragment thereof according to the present invention can reduce liver tropism originally possessed by the hepatocyte growth factor or the active fragment thereof and selectively exert the pharmacological effects of the hepatocyte growth factor or the active fragment thereof by exerting its bioactivity in a target disease tissue of the kidney or the like.

Brief Description of Drawings

[Figure 1] Figure 1 is a diagram showing the amounts of enzymatic activity exerted by ADAM 17 protein in kidney tissues and liver tissues of kidney disease model mice and normal mice.
[Figure 2] Figure 2 is a diagram showing the expression levels of ADAM17 protein in kidney tissues and liver tissues of kidney disease model mice and normal mice. Figure 2(a) shows a Western blotting image of the ADAM17 protein, and Figure 2(b) is a diagram showing a numerical values calculated from their band intensities.
[Figure 3] Figure 3 is a diagram showing the releases of the active form, human NK1, from the prodrugs of human NK1 by protease treatment.
[Figure 4] Figure 4 is a diagram showing the attenuations of the HGF activity of human NK1 by the prodrug modifications of protease-sensitive peptide-added human NK1.
[Figure 5] Figure 5 is a diagram showing the tissue-selective releases of the active form, human NK1, from the prodrugs of human NK1.
[Figure 6] Figure 6 is a diagram showing the *in vivo* tissue-selective bioactivity of the prodrug of human NK1.
[Figure 7] Figure 7 is a diagram showing the comparison of activity controls of the prodrugs of human NK1, depending on difference in polyethylene glycol modification site.

Description of Embodiments

<Polyethylene glycol-modified form of hepatocyte growth factor or active fragment thereof>

In the polyethylene glycol-modified form of a hepatocyte growth factor or an active fragment thereof (hereinafter, also referred to as the PEG-modified form of the HGF or an active fragment thereof) according to the present invention, polyethylene glycol(s) (hereinafter, also referred to as PEG) is/are covalently bound to (a) terminus(es) (amino terminus and/or carboxyl terminus) of one molecule of the hepatocyte growth factor (hereinafter, also referred to as HGF) or the active fragment thereof via (a) protease-sensitive peptide(s).

In one embodiment, examples of the PEG-modified form of the HGF or an active fragment thereof according to the present invention include a form in which one or more molecules of PEG are covalently bound to (a) terminus(es) (amino terminus or carboxyl terminus) of the HGF or the active fragment thereof via (a) protease-sensitive peptide(s). In this respect, the binding mode between the terminus(es) of the HGF or the active fragment thereof and the protease-sensitive peptide(s) is not particularly limited. They may be bound directly or may be arbitrarily bound via (a) spacer sequence(s). The binding mode between the protease-sensitive peptide(s) and the PEG is not particularly limited. The PEG may be bound directly to the protease-sensitive peptide(s) or may be arbitrarily bound thereto via an amino acid or the like artificially added to the protease-sensitive peptide(s). Further, a protease-sensitive peptide for purification or a tag sequence for purification may be contained between the protease-sensitive peptide and the artificially added amino acid or the like. Examples thereof include a form in which a terminus of the HGF or an active fragment thereof is covalently bound to the carboxyl terminus of a protease-sensitive peptide and the PEG is covalently bound to the amino terminus of the protease-sensitive peptide, and a form in which a terminus of the HGF or an active fragment thereof is covalently bound to the amino terminus of a protease-sensitive peptide and the PEG is covalently bound to the carboxyl terminus of the protease-sensitive peptide. A form is preferred in which a terminus of the HGF or an active fragment thereof is covalently bound to the carboxyl terminus of a protease-sensitive peptide and the PEG is covalently bound to the amino terminus of the protease-sensitive peptide.

The amino terminus of the HGF or the active fragment thereof is responsible for the functions of dimerization of the HGF or the active fragment thereof which are important for controlling the bioactivity of the HGF or the active fragment thereof, and is also responsible for liver tropism of the HGF or the active fragment thereof. Therefore, for reducing bioactivity and liver tropism possessed by the HGF or the active fragment thereof, a form is preferred in which one molecule of PEG is covalently bound to the amino terminus of the HGF or an active fragment thereof via a protease-sensitive peptide; a form is more preferred in which the carboxyl terminus of a protease-sensitive peptide is covalently bound to the amino terminus of the HGF or an active fragment thereof and further, one molecule of PEG is covalently bound to the amino terminus of the protease-sensitive peptide; a form is further preferred in which the carboxyl terminus of an ADAM17-sensitive peptide or a thrombin-sensitive peptide is covalently bound to the amino terminus of the HGF or an active fragment thereof and further, one molecule of PEG is covalently bound to the amino terminus of the ADAM17-sensitive peptide or the thrombin-sensitive peptide; a form is still further preferred in which the carboxyl terminus of an ADAM17-sensitive peptide or a thrombin-sensitive peptide is covalently bound to the amino terminus of NK1, an active fragment of HGF, and further, one molecule of PEG having a number-average molecular weight of 20000 to 100000 is covalently bound to the amino terminus of the ADAM17-sensitive peptide or the thrombin-sensitive peptide; and a form is most preferred in which the carboxyl terminus of an ADAM 17-sensitive peptide or a thrombin-sensitive peptide is covalently bound to the amino terminus of NK1 and further, one molecule of tetrabranched PEG having a number-average molecular weight of 70000 to 90000 is covalently bound to the amino terminus of the ADAM17-sensitive peptide or the thrombin-sensitive peptide. An arbitrary spacer sequence may be added to between the HGF or the active fragment thereof and the protease-sensitive peptide in order to control cleavage efficiency by protease.

In another embodiment, examples of the PEG-modified form of the HGF or an active fragment thereof according to the present invention also include a form in which one molecule of PEG is covalently bound to the middle of the sequence of the HGF or the active fragment thereof via a protease-sensitive peptide.

<HGF and active fragment thereof>

HGF is composed of N domain, kringle 1, kringle 2, kringle 3, kringle 4 and SPH domain in this order from the amino-terminal side. The N domain, kringle 1, kringle 2, kringle 3 and kringle 4 constitute an $\alpha$ chain, and the SPH domain constitutes a $\beta$ chain. HGF is biosynthesized as single-chain pro-HGF and secreted along with the removal of its secretory signal sequence. And then, the resulting sequence is extracellularly processed, between an $494^{th}$ arginine residue (R494) and a $495^{th}$ valine residue (V495) counted from initiating methionine, by protease to form a heterodimer chain of the $\alpha$ chain and the $\beta$ chain bound through a disulfide bond, which is active (Miyazawa K. et al., The Journal of Biological Chemistry, 1996, Vol. 271, No. 7, p. 3615-3618). In the present specification, the HGF means an active HGF having bioactivity.

It is known that diverse pharmacological effects of the HGF, such as a hepatocyte proliferative effect, an anti-apoptotic effect, an angiogenic effect, a vasodilatory effect, an anti-organ fibrosis effect, and an anti-epithelial mesenchymal transition effect, are induced by the binding of the HGF to c-Met, a receptor tyrosine kinase for the HGF. Through this binding of HGF to c-Met, a plurality of tyrosine residues on the intracellular side of the c-Met are phosphorylated, and signaling molecules bind to the phosphorylated tyrosine residues to activate the signaling pathway. In this respect, $1234^{th}$ and $1235^{th}$ tyrosine residues (Y1234/1235) are known as the particularly important phosphorylation sites.

Human HGF is biosynthesized as a secretory protein consisting of 728 amino acid residues (containing a secretory signal sequence (31 amino acid residues from initiating methionine)) (GenBank accession No: M29145) and, when secreted, becomes a protein consisting of 697 amino acid residues (SEQ ID NO: 1) by the removal of the secretory signal sequence.

The HGF described above encompasses a HGF having the same amino acid sequence as that of naturally occurring HGF (hereinafter, also referred to as natural HGF), as well as an amino acid mutant of HGF having an amino acid sequence derived from the amino acid sequence of natural HGF by the deletion, substitution or addition (or insertion) of one or several amino acids and having bioactivity as HGF, and further encompasses even a HGF having an altered sugar chain moiety of natural HGF and a HGF having no sugar chain moiety. The amino acid mutant of HGF is preferably a mutant having 90% or higher sequence identity to the amino acid sequence of natural HGF, more preferably a mutant having 95% or higher sequence identity thereto, further preferably a mutant having 98% or higher sequence identity. Examples of the amino acid mutant of HGF include a HGF with a deletion of 5 amino acid residues within kringle 1 (which is a naturally occurring mutant; hereinafter, also referred to as deleted variant of HGF) (Kinosaki M. et al., FEBS Letters, Vol. 434, 1998, p. 165-170), which has been reported to have higher specific activity than that of human HGF (SEQ ID NO: 1) in certain cell lines.

Conservative amino acid substitution generally refers to substitution between amino acids similar in chemical properties, electric properties (or polarity and/or hydrophobicity) or structural properties. Since such substitution can suppress a marked change in conformation of a polypeptide including natural HGF, the polypeptide can retain its activity without large impairment and, in sometimes, can have higher activity than the natural one. Specific examples of such amino acid substitution include substitution between acidic amino acids (e.g., aspartic acid (D) and glutamic acid (E)), substitution between basic amino acids (e.g., histidine (H), lysine (K) and arginine (R)), substitution between aromatic amino acids (e.g., phenylalanine (F), tyrosine (Y) and tryptophan (W)), substitution between hydrophilic amino acids (e.g., cysteine (C), aspartic acid (D), glutamic acid (E), histidine (H), lysine (K), asparagine (N), glutamine (Q), arginine (R), serine (S) and threonine (T)), and substitution between hydrophobic amino acids (e.g., alanine (A), phenylalanine (F), isoleucine (I), leucine (L), norleucine (Nle), methionine (M), valine (V), tryptophan (W) and tyrosine (Y)).

The HGF described above also encompasses a recombinant HGF prepared by a gene recombination technique on the basis of the amino acid sequence or nucleotide sequence of natural HGF.

The "sequence identity" used in the present specification refers to identity between two sequences that can be determined using an algorithm such as BLAST or FASTA, in which the two sequences are aligned so as to attain the maximum degree of identity with or without gaps involved, and can generally be calculated as the percentage (%) of the number of identical amino acids to the total number of amino acids (including gaps) (Altschul S. et al., Journal of Molecular Biology, 1990, Vol. 215, No. 3, p. 403-410; and Altschul S. et al., Nucleic Acids Research, 1997, Vol. 25, No. 17, p. 3389-3402).

The term "several" used in the present specification refers to an integer of 2 to 10, i.e., 2, 3, 4, 5, 6, 7, 8, 9 or 10.

The active fragment of HGF refers to a protein that has a portion of the structure of HGF and exerts bioactivity (agonistic activity) as HGF by binding to c-Met, or a protein that acts as an antagonist (exerts antagonistic activity) against c-Met.

Examples of the active fragment of HGF described above include NK1 and NK2 which are natural splicing variants of HGF, and NK4 developed by a recombination technique. NK1 is composed of N domain and kringle 1 on the amino-terminal side of HGF, and NK2 is composed of N domain, kringle 1 and kringle 2 on the amino-terminal side of HGF. It has been reported that each of these fragments acts as an agonist or an antagonist against c-Met *in vivo* (Jakubczak J.L. et al., Molecular and Cellular Biology, Vol. 18, No. 3, 1998, p. 1275-1283; and Otsuka T. et al., Molecular and Cellular Biology, Vol. 20, No. 6, 2000, p. 2055-2065). It has also been reported that NK1 has liver tropism and induces liver hypertrophy *in vivo* (Jakubczak J.L. et al., Molecular and Cellular Biology, 1998, Vol. 18, No. 3, p. 1275-1283; and Ross J. et al., Gastroenterology, 2012, Vol. 142, p. 897-906). NK4 is composed of N domain, kringle 1, kringle 2, kringle 3 and kringle 4 on the amino-terminal side of HGF and has been reported to act as an antagonist against c-Met (Date K. et al., FEBS Letters, Vol. 420, No. 1, 1997, p. 1-6).

The active fragment of HGF described above encompasses an active fragment having the same amino acid sequence as that derived from natural HGF, as well as a mutant having an amino acid sequence derived from the amino acid sequence of natural HGF by the deletion, substitution or addition of one or several amino acids and having bioactivity (agonistic activity) as HGF or antagonistic activity against c-Met, and further encompasses even a mutant having an altered sugar chain moiety derived from natural HGF and a mutant having no sugar chain moiety derived from natural HGF. The mutant of the active fragment of HGF is preferably a mutant having 90% or higher sequence identity to the amino acid sequence derived from natural HGF, more preferably a mutant having 95% or higher sequence identity thereto, further preferably a mutant having 98% or higher sequence identity. Examples of the highly active NK1 mutant include 1K1 (Lietha D. et al., The EMBO Journal, 2001, Vol. 20, No. 20, p. 5543-5555) and M2.2 (Jones D.S. 2nd. et al., Proceedings of the National Academy of Sciences of the United States of America, 2011, Vol. 108, No. 32, p. 13035-13040).

The HGF or the active fragment thereof contained in the PEG-modified form of HGF or an active fragment thereof according to the present invention is preferably natural HGF (also including a mutant such as deleted variant of HGF), NK1 (also including a mutant), NK2 (also including a mutant) or NK4 (also including a mutant), more preferably NK1 (also including a mutant) or NK2 (also including a mutant), further preferably NK1 (also including a mutant), most preferably human NK1 consisting of the amino acid sequence represented by SEQ ID NO: 2. The amino acid sequence represented by SEQ ID NO: 2 excludes a secretory signal sequence (MWVTKLLPALLLQHVLLHLLLLPIAIPYAEG: SEQ ID NO: 3) derived from human HGF.

The HGF or the active fragment thereof described above contains a mammal-derived amino acid sequence, which is preferably a human-, cat- or dog-derived amino acid sequence, more preferably a human-derived amino acid sequence.

The HGF or the active fragment thereof can be obtained, for example, according to a gene recombination technique known in the art by designing a nucleic acid sequence (DNA) encoding the HGF or the active fragment thereof, and transiently or stably introducing the nucleic acid sequence to cells for expression.

One to 20 amino acids may be added as a spacer sequence to between the HGF or the active fragment thereof and the protease-sensitive peptide by use of a gene recombination technique known in the art. The HGF or the active fragment thereof with the spacer sequence between the HGF or the active fragment thereof and the protease-sensitive peptide can be obtained, for example, according to a gene recombination technique known in the art by adding a nucleic acid (DNA) sequence encoding an arbitrarily selected spacer sequence to a nucleic acid (DNA) sequence encoding the HGF or the active fragment thereof so as to be consecutive, further adding a nucleic acid (DNA) sequence encoding the protease-sensitive peptide thereto so as to be consecutive, and transiently or stably introducing the resulting sequence to cells for expression. Examples of the amino acid that can be used in the spacer sequence include aspartic acid (D), glutamic acid (E), histidine (H), lysine (K), arginine (R), phenylalanine (F), tyrosine (Y), tryptophan (W), alanine (A), cysteine (C), asparagine (N), glutamine (Q), serine (S), threonine (T), isoleucine (I), leucine (L), norleucine (Nle), methionine (M), valine (V) and tryptophan (W). The spacer sequence used is appropriately selected in consideration of the cleavage efficiency of the protease-sensitive peptide, or the activity control efficiency of the HGF or the active fragment thereof by PEG modification.

<Protease-sensitive peptide>

The protease-sensitive peptide used in the present invention means a peptide that is cleaved by a protease whose expression is found in a target disease tissue other than a liver tissue and is relatively low in the liver tissue.

The protease corresponding to the protease-sensitive peptide needs to be selected as an appropriate one by evaluating a relative difference in enzymatic activity between the liver tissue and the target disease tissue by a method such as Western blotting or immunohistochemical staining, because a required level of the relative difference in enzymatic activity varies depending on the target disease tissue of interest. In one embodiment, when the target disease tissue is the kidney tissue, the protease needs to have 3-fold or more relative difference of enzymatic activity in the kidney tissue from that in the liver tissue and preferably have 10-fold or more such relative difference, more preferably have 100-fold or more such relative difference.

The PEG-modified form of the HGF or the active fragment thereof according to the present invention can be expected to reduce an adverse effect caused by the HGF or the active fragment thereof in the liver tissue, because a chemical modification with PEG can reduce the activity and liver tropism of the HGF or the active fragment thereof. On the other hand, a conjugation of the protease-sensitive peptide selected as above mentioned to the PEG-modified form of the HGF or the active fragment thereof may enable the HGF or the active fragment thereof to be released and exert its bioactivity in the target disease tissue when the protease-sensitive peptide is cleaved by its corresponding protease to liberate the PEG. A fragment of the protease-sensitive peptide cleaved by the corresponding protease may remain in the HGF or the active fragment thereof.

Examples of the protease whose expression is observed in the target disease tissue other than the liver tissue include ADAM17 (Melenhorst W.B. et al., American Journal of Physiology, 2009, Vol. 297, No. 3, p. 781-790), hepatocyte growth factor activator (HGFA), its upstream factor thrombin (David C.J.H. et al., American Journal of Physiology, 2001, Vol. 159, No. 4, p. 1383-1393) and matrix metalloprotease (Junwei Y. et al., The Journal of Clinical Investigation, 2002, Vol. 110, p. 1525-1538) which are reported to be expressed in the kidney of kidney disease patients.

ADAM17 is intracellularly synthesized as an inactive proform and then cleaved by furin into a mature form, which in turn exhibits an activity of cleaving various proteins (Aldo B. et al., Journal of Biological Chemistry, 2003, Vol. 278, p. 25933-25939; and Belen S.J. et al., Journal of Biological Chemistry, 2007, Vol. 282, p. 8325-8331). In the present specification, ADAM17 refers to mature ADAM17 unless otherwise specified.

An endogenous peptide which is previously reported to be cleaved by its corresponding protease, or an artificial peptide prepared by use of a gene recombination technique can be used as the protease-sensitive peptide contained in the PEG-modified form of the HGF or the active fragment thereof according to the present invention. The protease-sensitive peptide may be selected in consideration of a stability, cleavage efficiency in the target tissue and species cross-reactivity. The present inventors have found that the expressions of ADAM17 and thrombin in the liver tissue is relatively lower than that in the target disease tissue other than the liver tissue. Therefore, the protease-sensitive peptide is preferably an ADAM17-sensitive peptide or a thrombin-sensitive peptide. In this context, the ADAM17-sensitive peptide means a peptide that is cleaved by ADAM17, and the thrombin-sensitive peptide means a peptide that is cleaved by thrombin.

Examples of the endogenous peptide as the ADAM 17-sensitive peptide include a peptide derived from TNF-alpha (SPLAQAVRSSSR, SEQ ID NO: 8) and a peptide derived from L-selectin (QETNRSFSK, SEQ ID NO: 10). Examples of the artificial peptide include an artificial ADAM-17 cleavable sequence (PRAAAVKSP, SEQ ID NO: 9) (Caescu C.I. et al., Biochemical Journal, 2009, Vol. 424, p. 79-88). The ADAM17-sensitive peptide is preferably a peptide having the amino acid sequence represented by any of SEQ ID NOs: 8 to 16, more preferably a peptide having the amino acid sequence represented by SEQ ID NO: 9.

Examples of the endogenous peptide as the thrombin-sensitive peptide include a peptide derived from prothrombin (FNPRTFGS, SEQ ID NO: 19) and a peptide derived from HGFA (LRPRIIGG, SEQ ID NO: 20). Examples of the artificial peptide include an artificial thrombin cleavable sequence (TFLTPRGVRLG, SEQ ID NO: 17 and TFPPRSFLG, SEQ ID NO: 18) (Maike G. et al., PLoS ONE, 2012, Vol. 7, p. E31756-31771; and Bo C. et al., The Journal of Neuroscience, 2012, Vol. 32, No. 22, p. 7622-7631). The thrombin-sensitive peptide is preferably a peptide having the amino acid sequence represented by any of SEQ ID NOs: 17 to 20, more preferably a peptide having the amino acid sequence represented by SEQ ID NO: 17.

One or more protease-sensitive peptide sequences can be used. Use of the protease-sensitive peptides differing in cleavage reactivity to the same protease, or the protease-sensitive peptides for different proteases in combination allows a plurality of locations to be chemically modified with PEG or allows cleavage reactivity to be controlled.

The protease-sensitive peptide can be obtained as the protease-sensitive peptide-added HGF or active fragment thereof, for example, by use of a gene recombination technique known in the art by designing a nucleic acid (DNA) sequence encoding the protease-sensitive peptide sequence consecutively with a nucleic acid (DNA) sequence encoding the HGF or the active fragment thereof, and transiently or stably introducing the resulting sequence to cells for expression.

An artificial sequence such as a tag sequence may be added to the protease-sensitive peptide described above for the purpose of purification or the like. Examples of the tag sequence include a 6 × His tag, HAT tag, c-Myc tag, FLAG tag, DYKDDDDK tag, Strep tag, HA tag, GST tag and MBP tag. In addition to the protease-sensitive peptide, a sequence such as a protease-sensitive peptide for purification or a spacer sequence for purification may be arbitrarily inserted between the protease-sensitive peptide and such a tag sequence in order to remove the tag sequence after purification of the protease-sensitive peptide-added HGF or active fragment thereof. In this case, the protease-sensitive peptide-added HGF or active fragment thereof obtained by purification and the removal of the tag sequence may contain a cleaved fragment of the sequence such as the protease-sensitive peptide for purification or the spacer sequence for purification.

A natural amino acid such as cysteine or a non-natural amino acid such as azidophenylalanine may be inserted in the protease-sensitive peptide described above for the purpose of PEG modification or the like. The insertion of the amino acid above mentioned into the protease-sensitive peptide can be carried out, for example, by use of a gene recombination technique known in the art by designing a nucleic acid (DNA) sequence encoding the amino acid to be added consecutively with a nucleic acid (DNA) sequence encoding the protease-sensitive peptide sequence, and transiently or stably introducing the resulting sequence to cells for expression. The insertion site of the amino acid for the purpose of PEG modification or the like is preferably a terminus (amino terminus or carboxyl terminus) of the protease-sensitive peptide.

One to 20 amino acids may be added as a spacer sequence to between the protease-sensitive peptide and the artificial sequence such as the tag sequence for the purpose of purification or the like, or between the protease-sensitive peptide and the amino acid inserted for the purpose of PEG modification or the like, by use of a gene recombination technique known in the art. The protease-sensitive peptide with the spacer sequence between the protease-sensitive peptide and the artificial sequence such as the tag sequence for the purpose of purification or the like or the amino acid inserted for the purpose of PEG modification or the like can be obtained, for example, by use of a gene recombination technique known in the art by cloning a nucleic acid (DNA) sequence encoding the protease-sensitive peptide and adding a nucleic acid (DNA) sequence encoding an arbitrarily selected spacer sequence so as to be consecutive, and further inserting a nucleic acid (DNA) sequence encoding the artificial sequence such as the tag sequence for the purpose of purification or the like or the amino acid for the purpose of PEG modification or the like thereto so as to be consecutive, and transiently or stably introducing the resulting sequence to cells for expression. Examples of the amino acid that can be used in the spacer sequence include aspartic acid (D), glutamic acid (E), histidine (H), lysine (K), arginine (R), phenylalanine (F), tyrosine (Y), tryptophan (W), alanine (A), cysteine (C), asparagine (N), glutamine (Q), serine (S), threonine (T), isoleucine (I), leucine (L), norleucine (Nle), methionine (M), valine (V) and tryptophan (W). In this respect, the spacer sequence to be arbitrarily added is preferably added to a terminus of the protease-sensitive peptide. More preferably, the carboxyl terminus of the spacer sequence is added to the amino terminus of the protease-sensitive peptide added to the amino terminus of the HGF or the active fragment thereof, and the artificial sequence such as the tag sequence for the purpose of purification or the like or the amino acid inserted for the purpose of PEG modification or the like is added to the amino terminus of the spacer sequence. The spacer sequence used may be appropriately selected in consideration of the cleavage efficiency of the protease-sensitive peptide, or the control efficiency of the activity of the HGF or the active fragment thereof by PEG modification.

<PEG>

PEG is a highly biocompatible polymer including water-soluble poly(ethylene oxide). It is known that the chemical modification of a protein with PEG imparts clinical usefulness such as improvement in physical stability, heat stability, resistance to proteolytic enzymes, and solubility, decrease in *in vivo* distribution volume, and improvement in blood circulation to the protein (Inada et al., Journal of Bioact and Compatible Polymers, Vol. 5, 1990, p. 343; Delgado et al., Critical Reviews in Therapeutic Drug Carrier Systems, Vol. 9, 1992, p. 249; and Katre, Advanced Drug Delivery Systems, Vol. 10, 1993, p. 91).

PEG known in the art can be used for preparing the PEG-modified form of HGF or an active fragment thereof according to the present invention. Typically, the PEG has a repeat unit structure "$-(CH_2CH_2O)_n-$", and a terminal group or a whole structure of the PEG may vary as mentioned later. The PEG may contain, for example, "$-CH_2CH_2-O(CH_2CH_2O)_n-CH_2CH_2-$" and "$-(OCH_2CH_2)_nO-$", depending on the presence or absence of substitution of the terminal oxygen atom. The PEG that is used for preparing the PEG-modified form of the HGF or the active fragment thereof according to the present invention is terminally capped. Specific examples thereof include the PEG capped at one of its ends with a functional group having reactivity (e.g., a maleimide group). The other end of the PEG may be capped or may undergo, for example, modification such as the introduction of a hydroxyl group, in order to enhance hydrophilicity. In the case of using a PEG having a branched structure, at least one end can be capped with the functional group having reactivity, such as a maleimide group, and each of the other ends of the branched chain may undergo modification. In the present invention, the PEG activated with a functional group such that the PEG end exhibits covalent binding reactivity with the protease-sensitive peptide-added HGF or active fragment thereof can be used for chemically modifying the protease-sensitive peptide-added HGF or active fragment thereof with

PEG.

The PEG contained in the PEG-modified form of the HGF or the active fragment thereof according to the present invention is not particularly limited by its structure. The PEG may have a linear structure or a branched structure and preferably has a branched structure from the viewpoint of reducing the liver tropism of the HGF or the active fragment thereof. Examples of the PEG having a branched structure include dibranched PEG, tribranched PEG and tetrabranched PEG, though the number of branches also includes more than four. Among them, the tetrabranched PEG is preferred. More specific examples thereof include a structure dibranched, tribranched or tetrabranched from an end of a linker moiety up to the functional group, a structure where a PEG chain branch further arises from a PEG chain branch arising from an end of a linker moiety up to the functional group, and a structure where PEG chain branches arise from an end of a linker moiety up to the functional group and from the middle of the linker moiety. The PEG chains of the branches may have the same or different number-average molecular weights. Among others, a structure is preferred where two PEG chain branches arise from a linker moiety up to the functional group and two PEG chain branches further arise from the PEG chains of the branches, respectively; and a structure is most preferred where two PEG chain branches arise from a linker moiety up to the functional group and two PEG chain branches further arise from the PEG chains of the branches, respectively, wherein a total of the four PEG chains have the same number-average molecular weight. Examples of the PEG having the most preferred structure include SUNBRIGHT GL4-800MA from Yuka Sangyo Co., Ltd. (NOF Corp.). Preferred embodiments related to the structure of the PEG and preferred embodiments related to the number-average molecular weight of the PEG can be arbitrarily combined. Examples thereof include a tetrabranched PEG having a number-average molecular weight of 20000 to 100000, and a PEG having a number-average molecular weight of 70000 to 90000 and having a structure where two PEG chain branches arise from a linker moiety up to the functional group and two PEG chain branches further arise from the PEG chains of the branches, respectively, wherein the all of the four PEG chains have the same number-average molecular weight.

The PEG may be available as a commercial product, or can be synthesized by the ring-opening polymerization of ethylene oxide according to a method known in the art or a method equivalent thereto (Sandler and Karo, Polymer Synthesis, Academic Press, New York, Vol. 3, p. 138-161).

The number-average molecular weight of the PEG contained in the PEG-modified form of the HGF or the active fragment thereof according to the present invention is preferably 20000 to 100000, more preferably 40000 to 100000, further preferably 60000 to 100000, most preferably 70000 to 90000 for the attenuation of HGF activity. It is known that the *in vivo* half-life extending effect of chemical modification with PEG on a protein or the like correlates with the number-average molecular weight of the PEG (Sundqvist T. et al., Computers and Biomedical Research, 1988, Vol. 21, No. 2, p. 110-116). In general, PEG having a number-average molecular weight of 20000 or larger can be expected to have a sufficient *in vivo* half-life extending effect.

For chemically modifying the protease-sensitive peptide-added HGF or active fragment thereof with PEG, it is necessary to activate one terminus of the PEG. Examples of the activated PEG include PEG activated with a N-hydroxysuccinimide ester, nitrobenzenesulfonate ester, maleimide, o-pyridine disulfide, vinyl sulfone, iodoacetamide, carboxylic acid, azide, phosphine or amine structure at one terminus of the PEG. Such activated PEG may be a commercially available product, or can be synthesized according to a method known in the art. Examples of the PEG activated with each reactive functional group include SUNBRIGHT$^{(R)}$ (NOF Corp.) series from Yuka Sangyo Co., Ltd. In the present specification, the reactive functional group added to the PEG terminus so as to exhibit covalent binding reactivity with the protease-sensitive peptide-added HGF or active fragment thereof is simply referred to as a "functional group", and PEG having such a reactive functional group at its terminus is referred to as "PEG activated with the functional group". The functional group is appropriately selected depending on a modification site. For example, an amino group can be selectively modified by using a N-hydroxysuccinimide (NHS) ester group as the functional group. Also, a thiol group present in the side chain of a cysteine residue can be selectively modified by using a maleimide group as the functional group. Further, carboxyl groups present in the side chain of glutamic acid and the carboxyl terminus of protein can be modified using an amino group or the like. An azide group contained in a non-natural amino acid such as azidophenylalanine can be selectively modified using triallylphosphine. In these cases, the protease-sensitive peptide-added HGF or active fragment thereof modified with PEG, i.e., the PEG-modified form of HGF or an active fragment thereof according to the present invention, may contain a functional group resulting from the covalent binding reaction between the PEG and the protease-sensitive peptide, and contains, for example, a succinimide group resulted from the covalent binding reaction between a thiol group contained in cysteine and a maleimide group.

Preferred embodiments of the HGF or the active fragment thereof, preferred embodiments of the protease-sensitive peptide, and preferred embodiments of the PEG as described above can be arbitrarily combined.

<Production method>

In the PEG-modified form of the HGF or the active fragment thereof according to the present invention, as mentioned above, the PEG is/are covalently bound to (a) terminus(es) (amino terminus or carboxyl terminus) of the HGF or the active fragment thereof via (a) protease-sensitive peptide(s). Examples of the production method therefor include a method of covalently binding PEG to the protease-sensitive peptide-added HGF or active fragment thereof.

An exemplary method for producing the protease-sensitive peptide-added HGF or active fragment thereof will be described below. The HGF or the active fragment thereof and the protease-sensitive peptide can be expressed as one protein molecule without being separately prepared. The protease-sensitive peptide-added HGF or active fragment thereof can be obtained, for example, by use of a gene recombination technique mentioned later by designing a nucleic acid (DNA) sequence encoding the protease-sensitive peptide sequence consecutively with a nucleic acid (DNA) sequence encoding the HGF or the active fragment thereof, and transiently or stably introducing the resulting sequence to cells for expression. An arbitrary spacer sequence can also be added by designing a nucleic acid (DNA) sequence encoding the spacer sequence such that the spacer sequence is added consecutively between the HGF or the active fragment thereof and the protease-sensitive peptide, or between the protease-sensitive peptide and an artificial sequence such as a tag sequence for the purpose of purification or the like or an amino acid inserted for the purpose of PEG modification or the like.

In the production of the protease-sensitive peptide-added HGF or active fragment thereof, it is preferred to use the protease-sensitive peptide-added HGF or active fragment thereof obtained by expression in a protease-sensitive peptide-added state, for example, by use of a gene recombination technique. In this respect, the protease-sensitive peptide is preferably bound directly to the amino terminus of the HGF or the active fragment thereof. Preferably, the carboxyl terminus of the protease-sensitive peptide is bound directly to the amino terminus of the HGF or the active fragment thereof.

The purification or concentration of the protease-sensitive peptide-added HGF or active fragment thereof can be carried out by use of a method known in the art. The protease-sensitive peptide-added HGF or active fragment thereof can be purified or concentrated, for example, according to a method such as chromatography using ion exchange, gel filtration, a hydrophobic support or an affinity support, or a combination thereof by removing unreacted HGF or active fragment thereof or PEG activated with the functional group, or by-products.

The HGF or the active fragment thereof can also be obtained by use of a method known in the art, such as extraction from a tissue, protein synthesis using a gene recombination technique, or biological production using recombinant cells expressing the HGF or the active fragment thereof (or natural cells expressing the HGF). Alternatively, commercially available HGF or active fragment thereof may be used as the HGF or the active fragment thereof.

It is known that the HGF or an active fragment thereof can be expressed using prokaryotic cells or eukaryotic cells. For example, NK1 can be obtained by use of a gene recombination technique known in the art by transiently or stably introducing a nucleic acid (DNA) sequence encoding NK1, an active fragment of HGF, to cells for NK1 expression.

The gene recombination technique can be performed according to a method described in, for example, Molecular Cloning, 2nd ed., Cold Spring harbor Laboratory (1989). An exemplary such technique will be described below.

DNA encoding the HGF or the active fragment thereof is provided. The DNA can be obtained by isolating cDNA by selection from a cDNA library prepared from human tissues or cells, and subjecting the cDNA to a DNA amplification method such as PCR. Alternatively, the DNA can be chemically synthesized using, for example, a DNA synthesizer based on a phosphoramidite method.

The DNA described above is incorporated into an appropriate vector to prepare an expression vector. Such a vector contains elements, such as regulatory sequences, necessary for expressing the DNA (if necessary, and for secreting the protein). Specific examples of the elements include a translation start codon and stop codon, a promoter, an enhancer, a terminator, a ribosomal binding site (or a Shine-Dalgarno sequence), a selective marker sequence, and a signal sequence. The necessary elements are inserted in the vector.

A suitable promoter is selected as the promoter according to host cells. Examples of the promoter suitable for cells of *Escherichia* bacteria which are prokaryotes include trp promoter, lac promoter, recA promoter, and $\lambda P_L$ promoter. Examples of the promoter suitable for cells of *Bacillus* bacteria include SPO1 promoter, SPO2 promoter, and penP promoter. Examples of the promoter suitable for yeast cells include PHO5 promoter, PGK promoter, GAP promoter, ADH promoter, and AOX promoter. Examples of the promoter suitable for plant cells include cauliflower mosaic virus (CaMV) promoter. Examples of the promoter suitable for insect cells include P10 promoter and polyhedrin promoter. Examples of the promoter suitable for mammalian cells include promoters of viruses such as Rous sarcoma virus, polyoma virus, fowlpox virus, adenovirus, bovine papilloma virus, avian sarcoma virus, cytomegalovirus (SMV), simian virus 40 (SV40), and vaccinia virus, metallothionein promoter, and heat shock promoter.

Examples of the selective marker include HIS3 gene, LEU2 gene, TRP1 gene, URA3 gene, dihydrofolate reductase gene (methotrexate (MTX) resistance), ampicillin resistance gene, neomycin resistance gene, and kanamycin resistance gene.

A vector suitable for host cells is selected as the expression vector. For example, plasmids, phages, cosmids, virus vectors, and artificial chromosomes (e.g., BAC and YAC) are vectors usually used. Examples of the vector for a prokaryote include *E. coli*-derived plasmids, for example, plasmids of pCR series, plasmids of pBR series, and plasmids of pUC series, and *Bacillus subtilis*-derived plasmids, for example, pUB110, pTP5, and pC194. Examples of the vector for a yeast include yeast-derived plasmids, for example, plasmids of pSH series. Examples of the vector for a plant cell include binary vectors. Examples of the vector for a mammalian cell include commercially available vectors such as pBK-CMV, pcDNA3.1, and pZeoSV (Invitrogen Corp and Stratagene California), and virus vectors (e.g., vectors of adenovirus, adeno-associated virus, pox virus, herpes simplex virus, lentivirus, Sendai virus, vaccinia virus, and SV40).

Examples of the host cells include prokaryotic cells such as *E. coli* and *Bacillus subtilis,* and eukaryotic cells such as yeasts, plant cells, and animal cells (e.g., mammalian cells and insect cells). When the host cells are transformed or transfected with the expression vector, an approach known in the art can be used, for example, electroporation, microinjection, cell fusion, DEAE dextran method, calcium phosphate method, or particle gun method.

Examples of the method for binding the protease-sensitive peptide-added HGF or active fragment thereof to the PEG include a method of covalently binding the protease-sensitive peptide-added HGF or active fragment thereof described above to the PEG activated with the functional group, in other words, a method for chemically modifying the protease-sensitive peptide-added HGF or active fragment thereof with the PEG activated with the functional group.

For the chemical modification of the protease-sensitive peptide-added HGF or active fragment thereof described above with the PEG, an amino group, a thiol group, a carboxyl group or an azide group, etc. contained in an amino acid contained in the protease-sensitive peptide, or cysteine or a non-natural amino acid (e.g., azidophenylalanine) artificially introduced in the protease-sensitive peptide can be used. For the chemical modification of the protease-sensitive peptide-added HGF or active fragment thereof described above with the PEG, a gene recombination technique known in the art can be used (U.S. Patent No. 4917888, International Publication No. WO 1987/00056, International Publication No. WO 1999/55377, and Bioorganic & Medicinal Chemistry Letters, Vol. 14, 2004, p. 5743-5745). For example, in the case of artificially inserted cysteine into the protease-sensitive peptide added to the amino terminus or carboxyl terminus of the HGF or the active fragment thereof, a thiol group present in the side chain of the cysteine residue can be chemically modified in a site-selective manner with the PEG having a maleimide group as a functional group because the thiol group forms neither intramolecular nor intermolecular disulfide bonds. A method of introducing azidophenylalanine and azido-Z-lysine, etc. by codon alteration has been reported as a method for inserting a non-natural amino acid to the terminus of the protease-sensitive peptide in the protease-sensitive peptide-added HGF or active fragment thereof (JP Patent Publication (Kokai) No. 2009-207490 A (2009)). An azide group contained in such a non-natural amino acid may be chemically modified in a site-selective manner with the PEG having triallylphosphine as a functional group. Alternatively, the protease-sensitive peptide may be intracellularly labeled with biotin using Biotin-Ligase Expression System known in the art (Avidity LLC) and can be used as a site of chemical modification with the PEG through the use of interaction with avidin.

Among others, a method is preferred which involves artificially insertion of an amino acid (natural or non-natural) that exhibits a selective reactivity with the functional group to the terminus of the protease-sensitive peptide added to the terminus (amino terminus or carboxyl terminus) of the HGF or the active fragment thereof. The insertion of the amino acid (natural or non-natural) that exhibits selective reactivity with the functional group can be carried out by use of the gene recombination technique mentioned above. Particularly, for preparing the PEG-modified form of the HGF or the active fragment thereof according to the present invention, it is more preferred to artificially insert a cysteine residue, an azidophenylalanine residue or an azido-Z-lysine residue, and it is further preferred to artificially insert a cysteine residue. In this case, it is preferred to use the PEG activated with a maleimide group.

The purification or concentration of the PEG-modified form of the HGF or the active fragment thereof according to the present invention can be carried out by use of a method known in the art after chemical modification with the PEG via the protease-sensitive peptide. The PEG-modified form of the HGF or the active fragment thereof can be purified or concentrated, for example, according to a method such as chromatography using ion exchange, gel filtration, a hydrophobic support or an affinity support, or a combination thereof by removing unreacted HGF or active fragment thereof or PEG activated with the functional group, or by-products.

<Medicament>

The PEG-modified form of the HGF or the active fragment thereof according to the present invention can not only be expected to have an *in vivo* half-life extending effect owing to PEG modification but also can exert the bioactivity potentially possessed by the HGF or the active fragment thereof through the cleavage of the protease-sensitive peptide contained in the PEG-modified form in the target disease tissue expressing particular protease, and as such, can be used as an active ingredient for a medicament (e.g., a therapeutic or prophylactic drug for organ fibrosis such as renal fibrosis) with reduced adverse effects for the liver.

The bioactivity of the PEG-modified form of the HGF or the active fragment thereof described above can be conveniently measured using cultured cells. For example, a c-Met phosphorylation-inducing effect, a cell proliferative effect, a cell migration effect and an anti-apoptotic effect which are the pharmacological effects of the natural HGF can be used as indexes (Rubin J.S. et al, The Journal of Biological Chemistry, 2001, Vol. 276, No. 35, p. 32977-32983; Lietha D. et al., The EMBO Journal, 2001, Vol. 20, No. 20, p. 5543-5555; and Liu Y. et al., American Journal of Physiology, 1999, Vol. 277, No. 4, p. 624-633).

The release behavior of the active form, the HGF or the active fragment thereof, from the PEG-modified form of the HGF or the active fragment thereof described above can be conveniently measured, for example, by using SDS electrophoresis in which the molecular weight change between the PEG-modified form and the active form is measured as an index.

The *in vivo* half-life of the PEG-modified form of the HGF or the active fragment thereof described above can be calculated, for example, by measuring the concentration in blood of the PEG-modified form intravenously, intraperitoneally, subcutaneously or intradermally administered to experimental animals. Particularly, the radiolabeling of the PEG-modified form of the HGF or the active fragment thereof described above conveniently achieves such measurement.

The medicament comprising the PEG-modified form of HGF or an active fragment thereof described above as an active ingredient can be used in the treatment of a disease (acute inflammatory disease, chronic inflammatory disease, acute ischemic disease or chronic ischemic disease, etc.) that can exploit the pharmacological effects of the HGF or the active fragment thereof. The medicament can be used in the treatment of, for example, amyotrophic lateral sclerosis, organ fibrosis, diabetes mellitus, spinal cord injury, peritoneal adhesion or neuropathic pain as a specific disease, and further can be used for transplantation therapy, wound healing.

When the protease-sensitive peptide is, for example, an ADAM 17-sensitive peptide, the medicament comprising the PEG-modified form of the HGF or the active fragment thereof described above as an active ingredient can be used as a therapeutic or prophylactic agent for a disease (e.g., renal fibrosis) that develops in an organ (e.g., the kidney) having a higher expression level of ADAM17 than that of the liver in which an adverse effect of the HGF or the active fragment thereof is of concern. As for an organ having an equivalent or lower expression level of ADAM17 to or than that of the liver in healthy people, the medicament comprising the PEG-modified form of the HGF or the active fragment thereof described above as an active ingredient can also be used as a therapeutic agent for a disease as long as the disease is related to an organ in which the expression level of ADAM17 elevates due to a lesion and becomes higher than that of the liver.

The medicament described above can be used as a useful therapeutic drug for a mammal (e.g., a mouse, a rat, a hamster, a rabbit, a dog, a cat, a monkey, a cattle, a sheep or a human), particularly, a human. In the case of using the medicament described above as a useful therapeutic drug for a human, the HGF or the active fragment thereof described above is preferably based on an amino acid sequence derived from human HGF.

In the mode of administration of the medicament described above, the PEG-modified form of the HGF or the active fragment thereof serving as an active ingredient can be orally or parenterally administered, either alone or as a medicament blended with an acceptable carrier. The administration is preferably performed by subcutaneous, intramuscular or intravenous injection.

Examples of the dosage form for the oral administration of the medicament described above include tablets, pills, capsules, granules, syrups, emulsions and suspensions. These dosage forms can be produced by methods known in the art and contain a carrier or an excipient and an optional additive usually used in the pharmaceutical formulation field. Examples of the carrier and the excipient for tablets include lactose, maltose, saccharose, starch and magnesium stearate. Examples of the additive can include binders, disintegrants, preservatives, delayed releasing agents, colorants, flavoring agents, stabilizers, solubilizers, thickeners, and emulsifiers.

Examples of the dosage form for the parenteral administration of the medicament described above include injections, eye drops, ointments, wet dressings, suppositories, transnasal absorption agents, transpulmonary absorption agents, transdermal absorption agents and local sustained-release agents. These dosage forms can be produced by methods known in the art. Solution formulations can be prepared, for example, in a state where the PEG-modified form of the HGF or the active fragment thereof serving as an active ingredient is dissolved in an aseptic aqueous solution for use in an injection or suspended and emulsified in extracts, and embedded in liposomes. Solid formulations can be prepared, for example, as freeze-dried products of the PEG-modified form of the HGF or the active fragment thereof serving as an active ingredient supplemented with an excipient such as mannitol, trehalose, sorbitol, lactose or glucose. Such solid formulations may be further pulverized for use. Such powders may be mixed with polylactic acid, glycolic acid, or the like and solidified for use. Gels can be prepared, for example, by dissolving the PEG-modified form of the HGF or the active fragment thereof serving as an active ingredient in a thickener or a polysaccharide such as glycerin, PEG, methylcellulose, carboxymethylcellulose, hyaluronic acid or chondroitin sulfate. If necessary, these formulations can be supplemented with the additive described above.

The medicament described above can generally be administered at 0.001 mg to 100 mg/kg/dosage, preferably 0.01 mg to 10 mg/kg/dosage, in the range of once a month to once a day, preferably once a month to once a week, though the dose is appropriately determined according to the age and body weight of a patient, a disease to be administered, symptoms, the mode of administration, an administration route, the molecular weight of PEG, etc.

The medicament described above may be blended or used in combination in an appropriate amount with an additional drug in order to complement or enhance its therapeutic or prophylactic effect or to reduce its dose.

In another aspect, the PEG-modified form of the HGF or the active fragment thereof according to the present invention can be used as a cell differentiation-promoting or -inducing agent comprising the PEG-modified form of the HGF or the active fragment thereof through the use of the morphogenesis-inducing effect of the HGF or the active fragment thereof. Examples of the cell include epithelial cells and neural stem cells.

Examples

Hereinafter, the PEG-modified form of the HGF or the active fragment thereof according to the present invention will be specifically described with reference to Examples. However, the present invention is not limited by these examples.

(Example 1) Expression of protease-sensitive peptide-added human NK1:

DNA encoding the human NK1 in which the carboxyl terminus of the protease-sensitive peptide (SEQ ID NO: 9 as an ADAM17-sensitive peptide, SEQ ID NO: 17 as a thrombin-sensitive peptide) was added to the amino terminus of the human NK1, without its secretory signal sequence, and a 6 × His tag and a cysteine residue in this order were added to the amino terminus of the protease-sensitive peptide (i.e., CHHHHHH (SEQ ID NO: 4) were added to the protease-sensitive peptide-added human NK1) was prepared by artificial synthesis (FASMAC Co., Ltd.), and inserted to an expression vector Cold-shock promoter/pCold IV (Takara Bio Inc.). A host *E. coli* SHuffle T7 Express (New England Biolabs, Inc.) was transformed with the prepared plasmid for expression.

Transformation was also performed by a similar method with DNA encoding the human NK1 in which the amino terminus of the protease-sensitive peptide (SEQ ID NO: 9 as an ADAM17-sensitive peptide) was added to the carboxyl terminus of the human NK1, without its signal sequence, and a cysteine residue and 6 × His tag in this order were added to the carboxyl terminus of the protease-sensitive peptide (i.e., HHHHHHC (SEQ ID NO: 5) were added to the protease-sensitive peptide-added human NK1). Hereinafter, human NK1 to which the protease-sensitive peptide (i.e., the ADAM17-sensitive peptide or the thrombin-sensitive peptide) and the 6 × His tag and the cysteine residue represented by SEQ ID NO: 4 or 5 were added is collectively referred to as the protease-sensitive peptide-added human NK1.

The *E. coli* described above was spread over LB agar medium containing kanamycin sulfate or carbenicillin and cultured at 37°C for 18 to 22 hours for the first selective culture. The obtained colony was inoculated to LB medium containing kanamycin sulfate or carbenicillin and cultured for the second selective culture. This culture solution was used as a seed culture solution.

The seed culture solution was cultured in a culture solution containing kanamycin sulfate or carbenicillin, 0.8% glucose, 0.7% glycerol, 2% Bacto-Tryptone, 1% casamino acid, 1% yeast extract, 100 mmol/L potassium phosphate, 10 mmol/L $MgSO_4$ and 0.5% NaCl. For the culture, a glass flask or a mini jar fermenter was used. After confirmation that the turbidity of the culture solution elevated to an appropriate value, the culture solution was applied to cold shock at 15°C or lower. At the same time with the cold shock, IPTG was added to the culture solution. 1.5 to 3 hours after the cold shock, the culture was terminated, and the culture solution was harvested and centrifuged to obtain *E. coli* precipitates expressing the protease-sensitive peptide-added human NK1.

(Example 2) Purification of protease-sensitive peptide-added human NK1:

The protease-sensitive peptide-added human NK1 was purified by the following method from the *E. coli* precipitates obtained in Example 1.

The *E. coli* precipitates expressing the protease-sensitive peptide-added human NK1 obtained in Example 1 were suspended in 50 mmol/L Tris-HCl, 5 mmol/L EDTA, 150 mmol/L NaCl, 10 mmol/L $CaCl_2$, and 10 mmol/L $MgCl_2$ and disrupted using a high-pressure tabletop homogenizer (GEA Niro Soavi), and then, the homogenate was purified by centrifugation.

The centrifugally purified *E. coli* homogenate was passed through a heparin support (HiTrapHeparin; GE Healthcare Japan Corp.) equilibrated in advance with PBS(-) so that the protease-sensitive peptide-added human NK1 was bound to the heparin support. Subsequently, a buffer in which a concentration of NaCl contained in PBS(-) varied in the concentration range of 150 mmol/L to 2000 mmol/L was passed therethrough in the ascending order of the NaCl concentration to obtain each elution fraction. Each elution fraction was subjected to SDS electrophoresis to confirm an elution fraction of the protease-sensitive peptide-added human NK1.

Next, the elution fraction of the protease-sensitive peptide-added human NK1 obtained by heparin resin purification was passed through a nickel support (cOmplete$^{(R)}$ (Roche Diagnostics K.K.) His-Tag Purification Resin; Roche) equilibrated in advance with PBS(-) containing 300 mmol/L NaCl so that the protease-sensitive peptide-added human NK1 was bound to the resin. Subsequently, a buffer obtained by adding imidazole (concentration range: 0 mmol/L to 500 mmol/L) to PBS(-) containing 500 mM NaCl was passed therethrough in the ascending order of the imidazole concentration to obtain each elution fraction. Each elution fraction was subjected to SDS electrophoresis to confirm an elution fraction of the protease-sensitive peptide-added human NK1. The obtained elution fraction of the protease-sensitive peptide-added human NK1 was concentrated using a centrifugal ultrafiltration device (Amicon Ultra-15, MWCO = 10000; Merck Millipore) to obtain the protease-sensitive peptide-added human NK1.

(Example 3) Synthesis of prodrug of human NK1:

Human NK1 in which one molecule of PEG was covalently bound to the amino-terminal cysteine residue or carboxyl-terminal cysteine residue of one molecule of the protease-sensitive peptide-added human NK1 via the protease-sensitive peptide (hereinafter, also referred to as a prodrug of human NK1) was synthesized by the following method.

The vehicle of the protease-sensitive peptide-added human NK1 obtained by Example 2 was replaced with a 100 mmol/L phosphate buffer solution (pH 6.0) containing 300 mmol/L NaCl and 2 mmol/L EDTA, and PEG activated with the functional group was added to the protease-sensitive peptide-added human NK1 at a molar ratio of 40 times. The PEG was covalently bound to the protease-sensitive peptide-added human NK1 by overnight incubation at 25°C to synthesize a prodrug of human NK1. The PEG activated with the functional group used was SUNBRIGHT GL2-400MA (dibranched type, number-average molecular weight: 40000, Yuka Sangyo Co., Ltd.), SUNBRIGHT ME-400MA (linear type, number-average molecular weight: 40000, Yuka Sangyo Co., Ltd.) and SUNBRIGHT GL4-800MA (tetrabranched type, number-average molecular weight: 80000, Yuka Sangyo Co., Ltd.). The human NK1 with the ADAM17-sensitive peptide (SEQ ID NO: 9) added to the amino terminus was chemically modified with these three types of PEG (hereinafter, collectively referred to as an ADAM17-cleavable prodrug of human NK1, and described together with the structure and number-average molecular weight of the PEG used in the modification). The human NK1 with the ADAM17-sensitive peptide (SEQ ID NO: 9) added to the carboxyl terminus was chemically modified with the tetrabranched PEG having a number-average molecular weight of 80000. The human NK1 with the thrombin-sensitive peptide (SEQ ID NO: 17) added to the amino terminus was chemically modified with the dibranched PEG having a number-average molecular weight of 40000 (hereinafter, referred to as a thrombin-cleavable prodrug of human NK1).

Next, the reaction solution containing the prodrug of human NK1 was diluted 10-fold with NaCl-free PBS(-) and then passed through an ion-exchange support (SP-Sepharose 6 Fast Flow; GE Healthcare Japan Corp.) to remove unreacted PEG activated with the functional group from the solution. The prodrug of human NK1 adsorbed onto the ion-exchange support was eluted with PBS(-) containing 1.0 mol/L NaCl and then passed through a heparin support (Heparin-Sepharose Fast Flow; GE Healthcare Japan Corp.), and the flow-through solution was collected and concentrated using a centrifugal ultrafiltration device (Amicon Ultra-4, MWCO = 30000; Merck Millipore) to obtain a prodrug of human NK1 which was the protein of interest.

(Example 4) Measurement of purity of prodrug of human NK1 and contamination rate of protease-sensitive peptide-added human NK1:

The purity of the prodrug of human NK1 and the contamination rate of the precursor, the protease-sensitive peptide-added human NK1 having no chemical modification with PEG, were measured by reverse-phase chromatography using high-performance liquid chromatography.

A reverse-phase column (Intrada WP-RP; Imtakt Corp.) was connected to high-performance liquid chromatography (LC-10AD system; Shimadzu Corp.), and the ADAM17-cleavable prodrug of human NK1 (3 types) obtained in Example 3 was injected thereto. A peak was detected at a wavelength of 280 nm while the acetonitrile ratio of distilled water containing 0.1% trifluoroacetic acid (acetonitrile: 0%) was increased to 100% over time. A peak around a relative retention time of 0.65 minutes with respect to the prodrug of human NK1 that appeared as a main peak was regarded as the protease-sensitive peptide-added human NK1. The purity of the prodrug of human NK1 and the contamination rate of the protease-sensitive peptide-added human NK1 were calculated.

As a result, the purity of the ADAM17-cleavable prodrug of human NK1 chemically modified with the dibranched PEG having a number-average molecular weight of 40000 was 98.9% (contamination rate of the protease-sensitive peptide-added human NK1: 1.1%). The purity of the ADAM17-cleavable prodrug of human NK1 chemically modified with the linear PEG having a number-average molecular weight of 40000 was 97.3% (contamination rate of the protease-sensitive peptide-added human NK1: 2.7%). The purity of the ADAM17-cleavable prodrug of human NK1 chemically modified with the tetrabranched PEG having a number-average molecular weight of 80000 was 97.9% (contamination rate of the protease-sensitive peptide-added human NK1: 2.1%). Thus, it was confirmed that all the prodrugs of human NK1 were able to be obtained with high purity.

(Example 5) Measurement of amount of ADAM17 activity exerted in kidney disease model mouse tissue:

The amount of ADAM17 activity exerted in kidney disease model mouse tissues was measured using an ADAM17 activity measurement kit (SENSOLYTER 520 TACE; Anaspec Inc.). The measurement was performed in duplicate.

Mice with unilateral ureteral obstruction (hereinafter, referred to as UUO mice) were prepared as kidney disease model mice. The ureter of each male mouse of ICR strain (Charles River Laboratories Japan, Inc.) was ligated with a silk thread. The incision site was sutured. After raising for 1, 7, 10 and 14 days, the kidney and the liver of the mouse were harvested. Untreated mice (hereinafter, referred to as normal mice) were raised for 0 days and 14 days, and then, the kidney and the liver of each mouse were harvested. A vehicle obtained by adding 0.1% Triton-XlOO to Component C included in SENSOLYTER 520 TACE (Anaspec Inc.) was added to tissue pieces, and the tissue was disrupted using a handy microhomogenizer (Physcotron). The disrupted tissue was left standing at 4°C or on ice for 15 minutes and centrifuged at 2000 × g at 4°C for 15 minutes to obtain a supernatant.

The total protein level of the centrifugation supernatant was measured using Protein Assay (Bio-Rad Laboratories, Inc.) reagent.

The ADAM17 activity in the centrifugal supernatant was detected by measuring fluorescence at 535 nm upon excitation at 485 nm to calculate ADAM17 activity per μg of the protein.

The results are shown in Figure 1. The ordinate depicts the ADAM17 activity per μg of the protein in each sample. "Day 0", "Day 1", "Day 7", "Day 10" and "Day 14" on the abscissa represent the tissues of the mice raised for 0, 1, 7, 10, and 14 days, respectively. "Normal" represents the normal mice, and "UUO" represents the UUO mice.

Both the UUO mice and the normal mice exhibited ADAM17 activity in the kidney tissues exceeding that in the liver tissues. The activity of ADAM17 was increased with increase in the number of days after ureteral obstruction, indicating that the ADAM17 activity is increased in the kidney tissues with an advanced kidney disease as compared with the liver tissues and normal kidney tissues. Thus, ADAM17 was found to be useful as a target molecule for use in the activity control of the prodrug of the HGF or the active fragment thereof.

(Example 6) Measurement of ADAM17 protein expression level in kidney disease model mouse tissue:

The expression level of the ADAM17 protein in kidney disease model mouse tissues was evaluated by Western blotting. The evaluation was performed in duplicate.

The kidney and the liver were harvested from each of UUO mice prepared in the same way as in Example 5 on 7 and 14 days after ureteral obstruction for the UUO mice and normal mice on the day when the ureteral obstruction of the UUO mice was carried out for the normal mice (i.e., raised for 0 days). A vehicle obtained by adding 0.1% Triton-XlOO to Component C included in SENSOLYTER(R) (Anaspec Inc.) 520 TACE (Anaspec Inc.) was added to tissue pieces, and the tissue was disrupted using a handy microhomogenizer (Physcotron). The disrupted tissue was left standing at 4°C or on ice for 15 minutes and centrifuged at 2000 × g at 4°C for 15 minutes to obtain a supernatant.

Western blotting was performed using the obtained supernatant. An ADAM17 proform and mature ADAM17 were detected using Anti-ADAM17 antibody-Activation site (Abcam plc) as a primary antibody and anti-Rab IgG-HRP antibody (Cell Signaling Technology, Inc.) as a secondary antibody. GAPDH was detected using Human/Mouse/Rat GAPDH/G3PH Antibody (R&D Systems, Inc.) as a primary antibody and donkey anti goat IgG-HRP (Santa Cruz Biotechnology, Inc.) as a secondary antibody.

Images obtained with ChemiDoc(R) (Bio-Rad Laboratories, Inc.) XRS+ System (Bio-Rad Laboratories, Inc.) were analyzed. A value was calculated by dividing the band intensity of the ADAM17 proform or the mature ADAM17 by the band intensity of GAPDH, and used as an index for the expression level of the ADAM17 protein in the tissue.

The results are shown in Figure 2. Figure 2(a) shows the Western blotting image of each sample. Figure 2(b) shows a mean calculated from the expression level of the ADAM17 protein in the tissue calculated from the band intensity of each sample. "Day 0", "Day 7" and "Day 14" in the drawing represent the mice raised for 0, 7 and 14 days, respectively. "Normal" represents the normal mice, and "UUO" represents the UUO mice. The ordinate of Figure 2(b) depicts the expression level of the ADAM17 protein in the tissue.

Decrease in the expression level of the ADAM17 proform protein and increase in the expression level of the mature ADAM17 protein exhibiting ADAM17 activity were observed over time after ureteral obstruction in the kidney tissues.

These results demonstrated that the expression level of the mature ADAM17 protein is increased in the kidney tissues with an advanced kidney disease as compared with the liver tissues or normal kidney tissues.

Thus, ADAM17 was found to be useful as a target molecule for use in the activity control of the prodrug of the HGF or the active fragment thereof.

(Example 7) Evaluation of active form release by protease treatment of prodrug of human NK1:

The amount of human NK1 (active form) released by the protease treatment of the prodrug of human NK1 was evaluated by SDS electrophoresis.

Each prodrug of human NK1 (three ADAM17-cleavable prodrugs of human NK1 and one thrombin-cleavable prodrug of human NK1) obtained in Example 3 was diluted 10-fold with Component C included in SENSOLYTE 520 TACE (Anaspec Inc.). For the ADAM17-cleavable prodrug of human NK1, ADAM17 (TACE, His Tag, Human Recombinant; Calbiochem) was added at a reaction molar ratio of prodrug of human NK1:ADAM17 = 5:1. For the thrombin-cleavable prodrug of human NK1, thrombin (Thrombin from human plasma; Sigma-Aldrich Co. LLC) was added at a reaction molar ratio of prodrug of human NK1:thrombin = 1:10. The following operation was similarly performed on the prodrug of human NK1 non-supplemented with the corresponding protease as an experimental control. After incubation at 37°C for 1 hour, the reaction solution was subjected to TCA precipitation and SDS electrophoresis, and a molecular weight position of a band obtained by CBB staining was confirmed.

The results are shown in Figure 3. In the drawing, "-" represents a sample non-supplemented with protease, and "+" represents a sample supplemented with protease. "Thrombin-cleavable type" represents the thrombin-cleavable prodrug of human NK1, and "ADAM17-cleavable type" represents the ADAM17-cleavable prodrug of human NK1. The molecular weight refers to a number-average molecular weight.

The treatment of each prodrug of human NK1 with the corresponding protease (ADAM17 or thrombin) was found to cleave the protease-sensitive peptide contained in the prodrug of human NK1 so that the active form, human NK1, was released.

(Example 8) Evaluation of activity attenuation by chemical modification of NK1 with PEG:

The degree of attenuation of the HGF activity of the ADAM17-cleavable prodrug of human NK1 was evaluated by In-Cell ELISA using the amount of phosphorylation induced for HGF receptor present on the cell surface of a human lung epithelial cell line A549 as an index.

The A549 cells were suspended in MEM medium containing 10% FCS (Nacalai Tesque, Inc.), seeded at a density of $1.5 \times 10^4$ cells/well on a 96-well plate for imaging (Becton, Dickinson and Company), and cultured all night and all day. After reaching 70% confluency of the cells, the medium was replaced with serum-free MEM medium (Nacalai Tesque, Inc.), and the cells were cultured for 16 hours or longer into a serum-starved state. For the cells in the serum-starved state, the prodrug of human NK1 treated with ADAM17 or the prodrug of human NK1 untreated with ADAM17 in Example 7 was added, and reacted at 37°C for 10 minutes. In this Example, the prodrug of human NK1 used was the ADAM17-cleavable prodrug of NK1 (3 types) obtained by amino-terminal modification in Example 3.

The cells were fixed in PBS(-) containing 4% formalin. Subsequently, the cell membranes were permeabilized with PBS(-) containing 0.3% Triton-X and 0.6% hydrogen peroxide. Then, the cells were blocked with 10% BSA. For the cells thus blocked, anti-pYcMet antibody (Cell Signaling Technology, Inc.) as a primary antibody and anti-Rab IgG-HRP antibody (Cell Signaling Technology, Inc.) as a secondary antibody were added, and reacted, followed by the addition of 1 × QuantaRed Enhanced Chemifluorescent HRP Substrate (Thermo Fisher Scientific, Inc.). After incubation at room temperature, fluorescence intensity (RFU) at a wavelength of 590 nm was measured using a plate reader (Envision; PerkinElmer, Inc.) and used as the induced amount of HGF receptor phosphorylation.

Using the induced amount of HGF receptor phosphorylation, EC50 of a concentration reaction curve of the sample untreated with ADAM17 was calculated using Prism 4 (GraphPad, 4-parameter approximation). Next, a value (fold-change of an activity (hereinafter, referred to as y)) was calculated as the degree of HGF activity attenuation by PEG modification, by dividing the EC50 (nM) of the sample untreated with ADAM17 by a concentration (nM) of the sample treated with ADAM17 at which the same signal intensity as that for the EC50 value of the sample untreated with ADAM17 was attained.

Next, the CBB staining images obtained in Example 7 were analyzed using ChemiDoc XRS+ system (Bio-Rad Laboratories, Inc.), and the cleavage efficiency (hereinafter, referred to as w) of the prodrug of human NK1 treated with ADAM17 was calculated according to the formula (1) given below. The HGF activity (hereinafter, referred to as preNK1) of the protease-sensitive peptide-added human NK1 (i.e., the precursor contained in the sample non-supplemented with ADAM17 due to poor purification), the HGF activity (hereinafter, referred to as Pro) of the prodrug of human NK1, the HGF activity (hereinafter, referred to as Pro·cut) of the prodrug of human NK1 cleaved by ADAM17 contained in the sample supplemented with ADAM17, and the HGF activity (hereinafter, referred to as Pro·noncut) of the prodrug of human NK1 uncleaved by ADAM17 (i.e., the prodrug that remained without being cleaved) were defined according to the formulas (2) to (5), respectively, using the purity (%; hereinafter, referred to as v) of the prodrug of NK1 obtained in Example 4. In this respect, the relative activity (%) of the prodrug of NK1 was indicated by as (algebra) when the activity of the protease-sensitive peptide-added human NK1 was defined as 100.

Cleavage efficiency (%, w) of the prodrug of human NK1 = 100 - (Band intensity of the prodrug of human NK1 in the sample supplemented with ADAM17 / Band intensity of the prodrug of human NK1 in the sample non-supplemented with ADAM17) × 100 ... Formula (1)

Activity (preNK1) of the protease-sensitive peptide-added human NK1 contained in the sample non-supplemented with ADAM17 = (100 - v) / 100 × 1 ... Formula (2)

Activity (Pro) of the prodrug of human NK1 contained in the sample non-supplemented with ADAM17 = (v / 100) × (x / 100) ... Formula (3)

Activity (Pro·cut) of the cleaved prodrug of human NK1 contained in the sample supplemented with ADAM17 = (v / 100) × (w / 100) × 1 ... Formula (4)

Activity (Pro·noncut) of the uncleaved prodrug of human NK1 contained in the sample supplemented with ADAM17 = (v / 100) × (100 - w) / 100 × (x / 100) ... Formula (5)

In this context, the fold-change of the activity (y) can be described according to the formula (6) using the expressions defined according to the formulas (1) to (5).

Fold-change of the activity (y) = (preNK1 + Pro·cut + Pro·noncut) / (preNK1 + Pro) ... Formula (6)

The formula (6) was resolved as to algebra x to obtain the formula (7) given below. The actually measured values of v, w and y were substituted into the formula (7) to calculate the relative activity (%) x of the prodrug of NK1.

$$x = \{10^4 \times (100 - v) + 10^2 \times v \times w - 10^4 \times y \times (100 - v)\} / \{v \times (10^2 \times y) - v \times (100 - w)\}$$ ... Formula (7)

The results are shown in Figure 4. The ordinate depicts the fluorescence intensity (RFU) that indicates the induced amount of HGF receptor phosphorylation. The abscissa depicts the treatment concentration (nmol/L) of the prodrug of human NK1. In the drawing, "ADAM17-" represents the sample of the prodrug of human NK1 untreated with ADAM17, and "ADAM17+" represents the sample of the prodrug of human NK1 treated with ADAM17. "ADAM17-cleavable type" represents the ADAM17-cleavable prodrug of human NK1. The molecular weight refers to a number-average molecular weight.

Among all the ADAM17-cleavable prodrugs of human NK1 chemically modified with PEG, the prodrug of human NK1 untreated with ADAM17 (ADAM17-) exhibited lower activity than that of the prodrug of human NK1 treated with ADAM17 (ADAM17+), indicating that the HGF activity was attenuated due to the chemical modification with PEG. As compared with the protease-sensitive peptide-added human NK1 having no chemical modification with PEG, the HGF activity of the ADAM17-cleavable prodrug of human NK1 chemically modified with the dibranched PEG having a number-average molecular weight of 40000 was attenuated by 80.1%, the HGF activity of the ADAM17-cleavable prodrug of NK1 chemically modified with the linear PEG having a number-average molecular weight of 40000 was attenuated by 70.7%, and the HGF activity of the ADAM17-cleavable prodrug of NK1 chemically modified with the tetrabranched PEG having a number-average molecular weight of 80000 was attenuated by 97.1%. The HGF activity of the prodrug of human NK1 chemically modified with the tetrabranched PEG having a number-average molecular weight of 80000 among the three types of PEG was most attenuated and was 1/30 of the HGF activity of human NK1 released by ADAM17 treatment.

(Example 9) Evaluation of tissue-selective release of active form from prodrug of human NK1:

The amount of human NK1 (active form) released by the exposure of the prodrug of human NK1 to a tissue homogenate was evaluated by SDS electrophoresis. In this Example, the amino-terminally modified ADAM17-cleavable prodrug of human NK1 was used which was obtained by modification with the tetrabranched PEG having a number-average molecular weight of 80000 in Example 3. The evaluation was performed in duplicate or triplicate.

UUO mice were prepared in the same way as in Example 5 using male mice of C57BL/6J strain (Charles River Laboratories Japan, Inc.). After ureteral obstruction and raising for 16 days, the kidney and the liver were harvested from each of the UUO mice and normal mice.

To each of the harvested mouse kidney and liver, a buffer solution (pH 7) containing 50 mmol/L Tris-HCl, 10 mmol/L $CaCl_2$ and 0.05% Brij35 was added, and a supernatant of a tissue homogenate was obtained in the same way as in Example 6. The protein level in the supernatant of the tissue homogenate was measured using Protein Assay (Bio-Rad Laboratories, Inc.).

The ADAM17-cleavable prodrug of human NK1 was added to the supernatant of each tissue homogenate, and incubated at 37°C for 30 minutes.

Western blotting was performed using the sample incubated above. HGF$\alpha$ (B-3) mouse monoclonal IgG (Santa Cruz Biotechnology, Inc.) was used as a primary antibody, and Peroxidase-conjugate AffiniPure Donkey Anti-Mouse IgG (Jackson ImmunoResearch Laboratories, Inc.) was used as a secondary antibody. Since the band of the prodrug of NK1 was not able to be detected by this method, bands of the protease-sensitive peptide-added human NK1 (i.e., the precursor contaminated due to a poor purification) contained in the prodrug of human NK1 and human NK1 released from the ADAM17-cleavable prodrug of human NK1 were detected and used as an index in evaluation.

The Western blotting image of each sample is shown in Figure 5. The band of the active form, human NK1, was detected in the sample of the ADAM17-cleavable prodrug of human NK1 admixed with the kidney homogenate, and its band intensity was higher than that of the protease-sensitive peptide-added human NK1 (i.e., the precursor contaminated due to a poor purification) contained in the prodrug of human NK1, indicating that this human NK1 was the active form released from the prodrug of human NK1. The band intensity of human NK1 was higher in the UUO kidney homogenate having high ADAM17 activity, indicating that a larger amount of human NK1 was released. By contrast, no band of the active form human NK1 was detected in the ADAM17-cleavable prodrug of human NK1 admixed with the liver homogenate. Thus, the ADAM17-cleavable prodrug of human NK1 was found to release the active form, human NK1, in a kidney tissue-selective manner.

Thus, the ADAM 17-cleavable prodrug of human NK1 was found to reduce HGF activity in the liver tissues, exhibit higher HGF activity in highly ADAM17-expressing kidney tissues than that in the liver tissues, be able to be expected to have drug efficacy in the kidney tissues, and be capable of reducing adverse effects in the liver tissues.

(Example 10) *In vivo* activity measurement of prodrug of human NK1:

The *in vivo* tissue-selective induced amount of c-Met phosphorylation by the ADAM17-cleavable prodrug of human NK1 was evaluated by Western blotting using tissue homogenates of mice given the ADAM17-cleavable prodrug of human NK1 or the protease (ADAM17)-sensitive peptide-added human NK1. In this Example, the amino-terminally modified ADAM17-cleavable prodrug of human NK1 was used which was obtained by chemical modification with the dibranched PEG having a number-average molecular weight of 40000 in Example 3, and also, the protease (ADAM17)-sensitive peptide-added human NK1 was used which was obtained in Example 2.

UUO mice were prepared in the same way as in Example 5. Fourteen days after ureteral obstruction, the protease (ADAM17)-sensitive peptide-added human NK1 (PEG-unmodified) or the prodrug of human NK1 having the dibranched PEG having a number-average molecular weight of 40000 was administered at a dose of 400 $\mu$g/kg from the tail vein. The following operation was similarly performed on the normal mice and UUO mice given a vehicle (PBS(-) containing 0.3 mol/L NaCl) for the prodrug of human NK1 as an experimental control. The kidney and the liver were harvested from each mouse at 0.18 hours after administration.

The mouse kidney and liver were each disrupted using a tabletop multi-sample cell disruption apparatus (Shake Master Neo; Bio-Medical Science Co., Ltd.). RIPA Buffer supplemented with a phosphatase inhibitor and a protease inhibitor was added to each homogenate, and the mixture was left standing on ice for 2 hours and then centrifuged to obtain a supernatant. The protein level was measured in the same way as in Example 8.

Western blotting was performed using the supernatant. Total c-Met was detected using anti-total cMet antibody (Cell Signaling Technology, Inc.) as a primary antibody and anti-Ms IgG-HRP antibody (Jackson ImmunoResearch Laboratories, Inc.) as a secondary antibody. Phosphorylated c-Met was detected using anti-pYcMet antibody (Cell Signaling Technology, Inc.) as a primary antibody and anti-Rab IgG-HRP antibody (Cell Signaling Technology, Inc.) as a secondary antibody.

Images taken using ChemiDoc XRS+ system (Bio-Rad Laboratories, Inc.) were analyzed. A value was calculated by dividing the band intensity of phosphorylated c-Met by the band intensity of Total c-Met. From the calculated value, a relative value was calculated with the value in the kidney of the UUO mouse of vehicle administration group defined as 1, and used as an index for the induced amount of HGF receptor phosphorylation in evaluation.

The results are shown in Figure 6. The ordinate depicts the value of the induced amount of HGF receptor phosphorylation. On the abscissa, "Normal + Vehicle" represents the vehicle administration group of the normal mice, "UUO + Vehicle" represents the vehicle administration group of the UUO mice, "UUO + Protease-sensitive peptide-added human NK1" represents the protease-sensitive peptide-added human NK1 administration group of the UUO mice, and "UUO + Prodrug of human NK1" represents the prodrug-of-human NK1 administration group of the UUO mice.

In the UUO mouse kidney tissues, the induced amount of HGF receptor phosphorylation was equivalent between the protease-sensitive peptide-added human NK1 administration group and the prodrug-of-human NK1 administration group. By contrast, for the liver tissues, the induced amount of HGF receptor phosphorylation by the prodrug of human NK1 was attenuated by 69% as compared with the protease-sensitive peptide-added human NK1. Thus, the prodrug of human NK1 was found to selectively exert the bioactivity in a target disease tissue (i.e., a kidney tissue highly expressing ADAM17) *in vivo* and reduce the liver tropism potentially possessed by the protease-sensitive peptide-added human NK1.

(Example 11) Comparison of activity control of prodrug of human NK1 depending on difference in PEG modification site:

Influence on HGF activity control depending on a difference in the positions of PEG modification of the protease-sensitive peptide-added human NK1 with the ADAM17-sensitive peptide added to the amino terminus obtained in Example 2 was evaluated by In-Cell ELISA using the induced amount of phosphorylation of HGF receptor present on the cell surface of a human lung epithelial cell line A549 as an index. In this Example, the ADAM17-cleavable prodrug of human NK1 was used which was obtained by the chemical modification of the amino terminus of human NK1 with the dibranched PEG having a number-average molecular weight of 40000 via the ADAM 17-sensitive peptide (SEQ ID NO: 9) in Example 3 (hereinafter, referred to as amino-terminally modified prodrug of human NK1), and also, the ADAM17-cleavable prodrug of human NK1 was used which was obtained by the chemical modification of the carboxyl terminus of human NK1 with the dibranched PEG having a number-average molecular weight of 40000 via the ADAM17-sensitive peptide (SEQ ID NO: 9) in Example 3 (hereinafter, referred to as carboxyl-terminally modified prodrug of human NK1).

The A549 cells were suspended in MEM medium containing 10% FCS (Nacalai Tesque, Inc.), seeded at a density of $1.5 \times 10^4$ cells/well to a 96-well plate for imaging (Becton, Dickinson and Company), and cultured all night and all day. After reaching 70% confluency of the cells, the medium was replaced with serum-free MEM medium (Nacalai Tesque, Inc.), and the cells were cultured for 16 hours or longer into a serum-starved state. For the cells in the serum-starved state, the prodrug of human NK1 or the protease-sensitive peptide-added human NK1 was added, and reacted at 37°C for 10 minutes.

The cells were fixed in PBS(-) containing 4% formalin. Subsequently, the cell membranes were permeabilized with PBS(-) containing 0.3% Triton-X and 0.6% hydrogen peroxide. Then, the cells were blocked with 10% BSA. To the cells thus blocked, anti-pYcMet antibody (Cell Signaling Technology, Inc.) as a primary antibody and anti-Rab IgG-HRP antibody (Cell Signaling Technology, Inc.) as a secondary antibody were added, and reacted, followed by the addition of $1 \times$ QuantaRed Enhanced Chemifluorescent HRP Substrate (Thermo Fisher Scientific, Inc.). After incubation at room temperature, fluorescence intensity (RFU) at a wavelength of 590 nm was measured using a plate reader (Envision; PerkinElmer, Inc.) and used as the induced amount of HGF receptor phosphorylation.

Using the induced amount of HGF receptor phosphorylation, a relative value for each prodrug of human NK1 with the protease-sensitive peptide and PEG added to the amino terminus or the carboxyl terminus of human NK1 was calculated when the value for the corresponding protease-sensitive peptide-added human NK1 unmodified with PEG was defined as 100.

The results are shown in Figure 7. The ordinate depicts the value of the induced amount of HGF receptor phosphorylation, which is indicated as a relative value when the value from the treatment with the corresponding protease-sensitive peptide-added human NK1 is defined as 100. The legend "Amino-terminal modification" represents the sample in which the protease-sensitive peptide and PEG were added to the amino terminus of the human NK1, and the legend "Carboxyl-terminal modification" represents the sample in which the protease-sensitive peptide and PEG were added to the carboxyl terminus of the human NK1.

The induced amount of HGF receptor phosphorylation (relative value) by the prodrug of human NK1 was 41 for the amino-terminally modified prodrug of human NK1 and by contrast, was 58 for the carboxyl-terminally modified prodrug of human NK1. The activity of the amino-terminally modified prodrug of human NK1 was more attenuated by chemical modification with PEG as compared with the carboxyl-terminally modified prodrug of human NK1. Thus, the PEG-modified form of HGF or an active fragment thereof according to the present invention was found to be able to more attenuate the activity of the HGF or the active fragment thereof through modification on the amino terminus thereof.

Industrial Applicability

The polyethylene glycol-modified form of a hepatocyte growth factor or an active fragment thereof according to the present invention can selectively release the hepatocyte growth factor or the active fragment thereof in a target disease tissue and therefore exhibits high activity in the target disease tissue, while the polyethylene glycol-modified form can be used as a medicament with reduced adverse effects caused by the hepatocyte growth factor or the active fragment thereof in liver tissues.

Free Text of Sequence Listing

SEQ ID NO: 1: Amino acid sequence of human HGF containing no secretory signal sequence
SEQ ID NO: 2: Amino acid sequence of human NK1 containing no secretory signal sequence
SEQ ID NO: 3: Secretory signal sequence derived from human HGF
SEQ ID NO: 4: Amino acid sequence of a cysteine residue and 6 × His tag added to the amino terminus of human NK1
SEQ ID NO: 5: Amino acid sequence of 6 × His tag and a cysteine residue added to the carboxyl terminus of human NK1
SEQ ID NO: 6: Nucleotide sequence of human HGF gene
SEQ ID NO: 7: Amino acid sequence of human HGF
SEQ ID NOs: 8 to 16: Amino acid sequence of an ADAM17-sensitive peptide
SEQ ID NOs: 17 to 20: Amino acid sequence of a thrombin-sensitive peptide

## Claims

1. A polyethylene glycol-modified form of a hepatocyte growth factor or an active fragment thereof, wherein the polyethylene glycol(s) is/are bound to any end of the hepatocyte growth factor or the active fragment thereof via (a) protease-sensitive peptide(s).

**2.** The polyethylene glycol-modified form of a hepatocyte growth factor or an active fragment thereof according to claim 1, wherein the protease-sensitive peptide(s) is/are (an) ADAM17-sensitive peptide(s) or (a) thrombin-sensitive peptide(s).

**3.** The polyethylene glycol-modified form of a hepatocyte growth factor or an active fragment thereof according to claim 1, wherein the protease-sensitive peptide(s) has/have an amino acid sequence represented by any one of SEQ ID NOs: 8 to 20 in the sequence listing.

**4.** The polyethylene glycol-modified form of a hepatocyte growth factor or an active fragment thereof according to any one of claims 1 to 3, wherein (a) number-average molecular weight(s) of the polyethylene glycol(s) is/are 20000 to 100000.

**5.** The polyethylene glycol-modified form of a hepatocyte growth factor or an active fragment thereof according to any one of claims 1 to 4, wherein the active fragment of the hepatocyte growth factor is NK1.

**6.** The polyethylene glycol-modified form of a hepatocyte growth factor or an active fragment thereof according to any one of claims 1 to 5, wherein the active fragment of the hepatocyte growth factor has the amino acid sequence represented by SEQ ID NO: 2 in the sequence listing.

**7.** A medicament comprising a polyethylene glycol-modified form of a hepatocyte growth factor or an active fragment thereof according to any one of claims 1 to 6 as an active ingredient.

Fig. 1

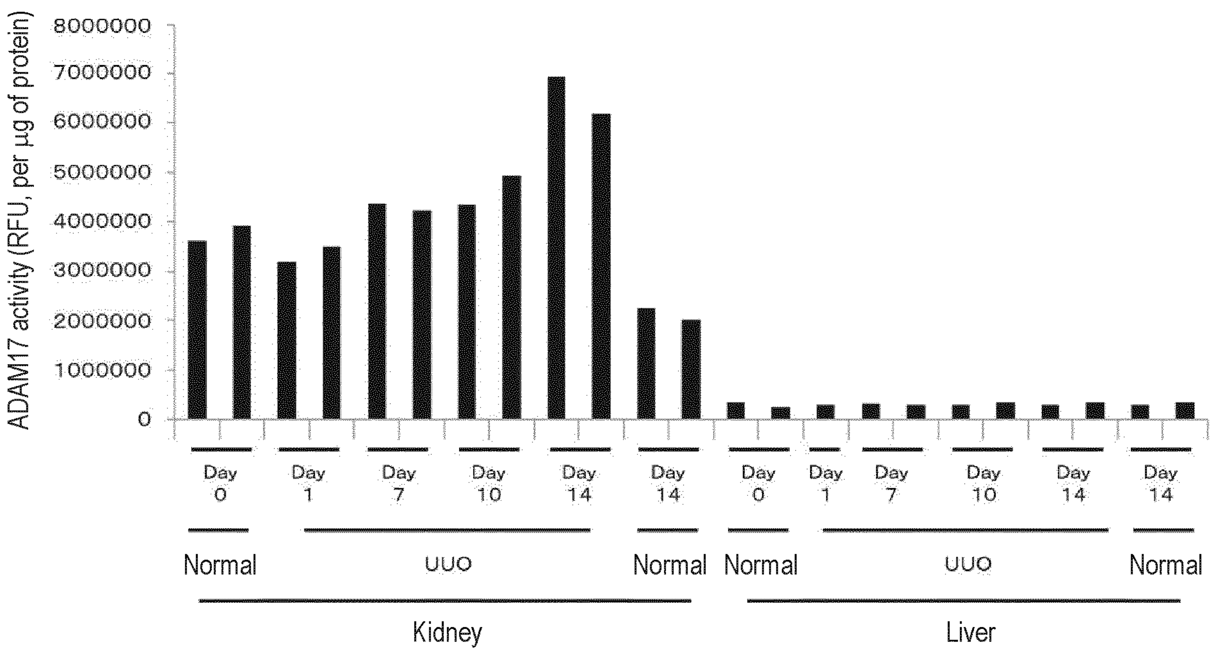
ADAM17 activity (RFU, per µg of protein)
8000000
7000000
6000000
5000000
4000000
3000000
2000000
1000000
0
Day 0
Day 1
Day 7
Day 10
Day 14
Day 14
Day 0
Day 1
Day 7
Day 10
Day 14
Day 14
Normal
UUO
Normal
Normal
UUO
Normal
Kidney
Liver

Fig. 2

(a)

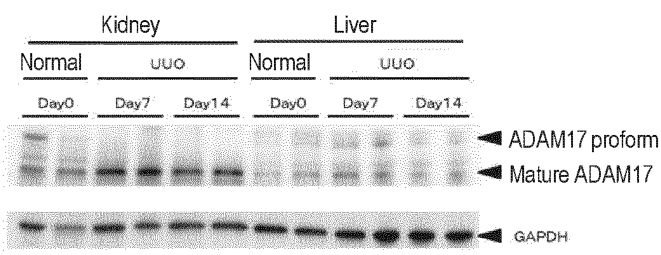
Kidney
Liver
Normal
UUO
Normal
UUO
Day0
Day7
Day14
Day0
Day7
Day14
ADAM17 proform
Mature ADAM17
GAPDH

(b)

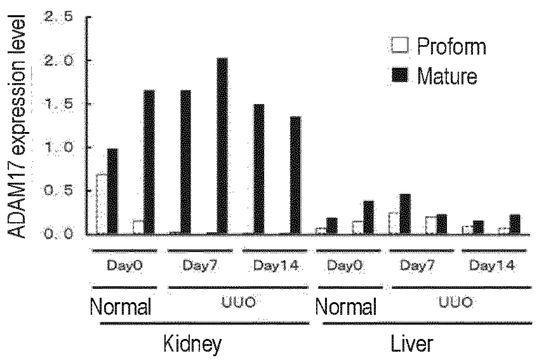
ADAM17 expression level
2.5
2.0
1.5
1.0
0.5
0.0
Proform
Mature
Day0
Day7
Day14
Day0
Day7
Day14
Normal
UUO
Normal
UUO
Kidney
Liver

Fig. 3

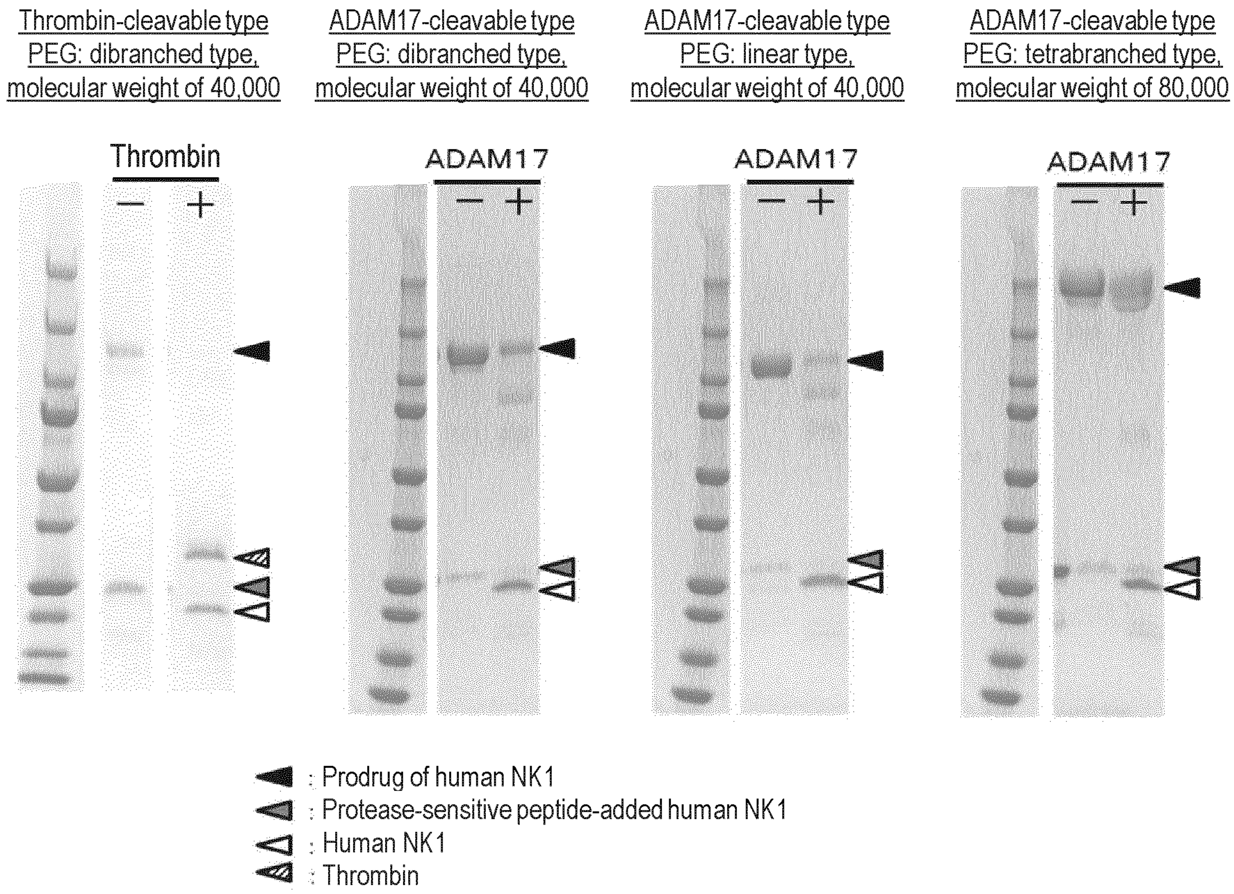
Thrombin-cleavable type
PEG: dibranched type,
molecular weight of 40,000
ADAM17-cleavable type
PEG: dibranched type,
molecular weight of 40,000
ADAM17-cleavable type
PEG: linear type,
molecular weight of 40,000
ADAM17-cleavable type
PEG: tetrabranched type,
molecular weight of 80,000
Thrombin
− +
ADAM17
− +
ADAM17
− +
ADAM17
− +
: Prodrug of human NK1
: Protease-sensitive peptide-added human NK1
: Human NK1
: Thrombin

# Fig. 4

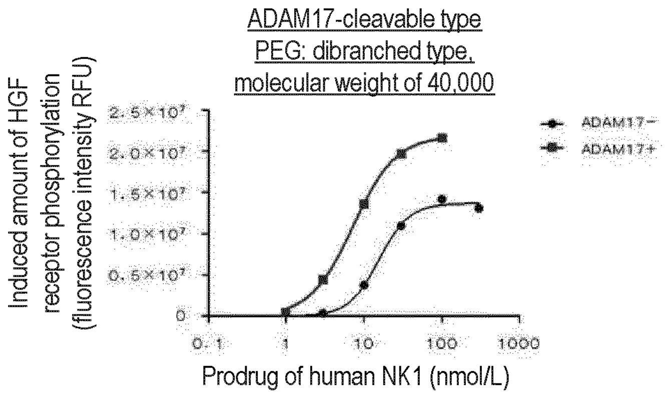


Contamination rate of protease-sensitive peptide-added human NK1: 1.1%

Cleavage efficiency by ADAM17: 66.1%

| | HGF activity |
|---|---|
| Protease-sensitive peptide-added human NK1 | 100. 0 |
| Prodrug of human NK1 | 19. 9 |

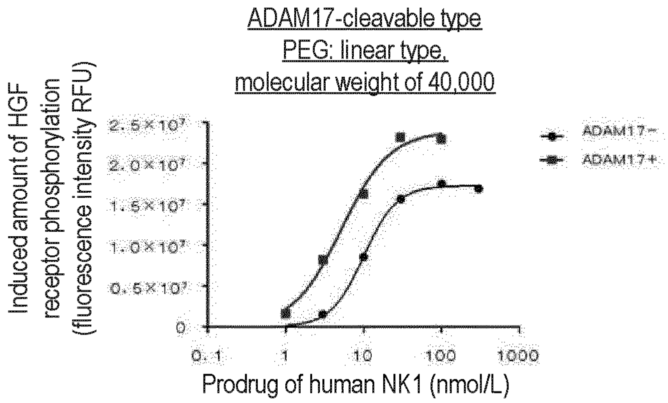


Contamination rate of protease-sensitive peptide-added human NK1: 2.7%

Cleavage efficiency by ADAM17: 85.6%

| | HGF activity |
|---|---|
| Protease-sensitive peptide-added human NK1 | 100. 0 |
| Prodrug of human NK1 | 29. 3 |

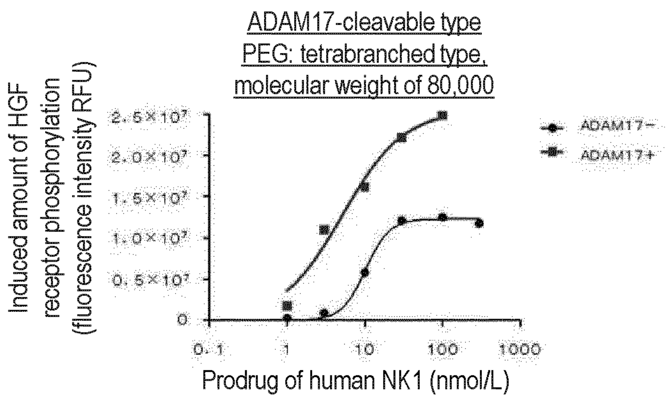


Contamination rate of protease-sensitive peptide-added human NK1: 2.1%

Cleavage efficiency by ADAM17: 24.3%

| | HGF activity |
|---|---|
| Protease-sensitive peptide-added human NK1 | 100. 0 |
| Prodrug of human NK1 | 2. 9 |

Fig. 5

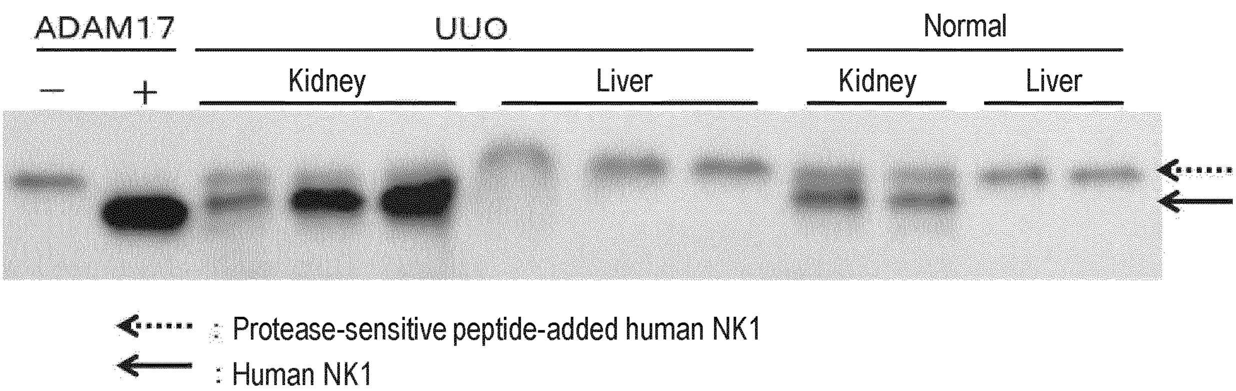
ADAM17
UUO
Normal
−
+
Kidney
Liver
Kidney
Liver
: Protease-sensitive peptide-added human NK1
: Human NK1

Fig. 6

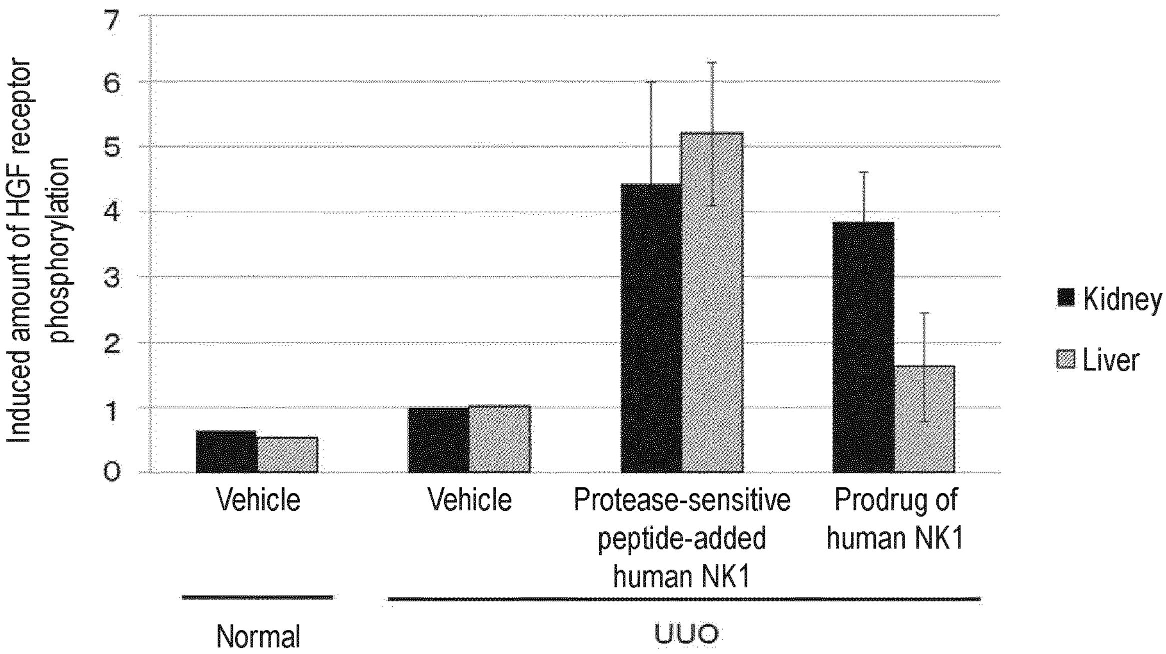
Induced amount of HGF receptor phosphorylation
7
6
5
4
3
2
1
0
Vehicle
Vehicle
Protease-sensitive peptide-added human NK1
Prodrug of human NK1
Normal
UUO
Kidney
Liver

Fig. 7

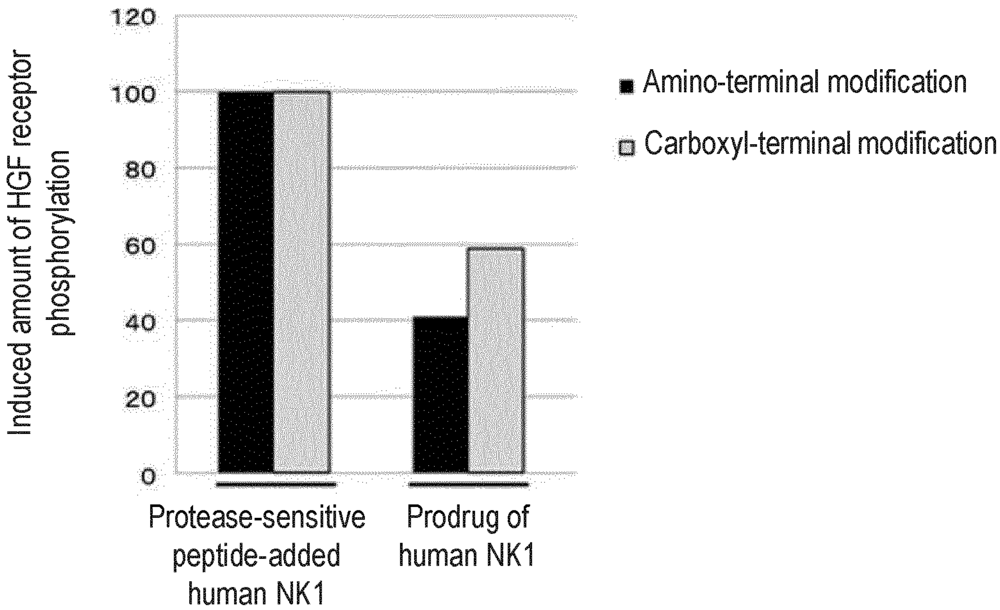
Induced amount of HGF receptor phosphorylation
120
100
80
60
40
20
0
Amino-terminal modification
Carboxyl-terminal modification
Protease-sensitive peptide-added human NK1
Prodrug of human NK1

## INTERNATIONAL SEARCH REPORT

International application No.
PCT/JP2020/002864

A. CLASSIFICATION OF SUBJECT MATTER
A61P 43/00(2006.01)i; C07K 14/475(2006.01)i; C07K 17/10(2006.01)i; C07K 19/00(2006.01)i; A61K 47/60(2017.01)i; A61K 47/65(2017.01)i; A61K 8/18(2006.01)i
FI: C07K14/475 ZNA; A61K38/18; A61K47/60; A61K47/65; A61P43/00 111; C07K17/10; C07K19/00

According to International Patent Classification (IPC) or to both national classification and IPC

B. FIELDS SEARCHED

Minimum documentation searched (classification system followed by classification symbols)
A61P43/00; C07K14/475; C07K17/10; C07K19/00; A61K47/60; A61K47/65; A61K38/18

Documentation searched other than minimum documentation to the extent that such documents are included in the fields searched

| | |
|---|---|
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)
JSTPlus/JMEDPlus/JST7580(JDream III); Caplus/MEDLINE/BIOSIS/WPIDS (STN)

C. DOCUMENTS CONSIDERED TO BE RELEVANT

| Category* | Citation of document, with indication, where appropriate, of the relevant passages | Relevant to claim No. |
|---|---|---|
| A | WO 96/28475 A1 (NAKAMURA, Toshikazu, SUMITOMO PHARMACEUTICALS CO., LTD.) 19.09.1996 (1996-09-19) claims | 1-7 |
| A | WO 02/074344 A2 (F. HOFFMANN-LA ROCHE AG) 26.09.2002 (2002-09-26) claims | 1-7 |
| A | STEFAN, N. et al., "Novel prodrug-like fusion toxin with protease-sensitive bioorthogonal PEGylation for tumor targeting", Bioconjug. Chem., 28 October 2014, vol. 25, no. 12, pp. 2144-2156, abstract | 1-7 |

☐ Further documents are listed in the continuation of Box C. ☒ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 07 April 2020 (07.04.2020) | 14 April 2020 (14.04.2020) |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

**INTERNATIONAL SEARCH REPORT**
Information on patent family members

International application No.
PCT/JP2020/002864

| Patent Documents referred in the Report | Publication Date | Patent Family | Publication Date |
|---|---|---|---|
| WO 96/28475 A1 | 19 Sep. 1996 | JP 4067058 B2 claims<br>US 5977310 A claims<br>EP 816381 A1 claims | |
| WO 02/074344 A2 | 26 Sep. 2002 | JP 2004-521139 A claims<br>US 2003/0012775 A1 claims<br>EP 1234583 A1 claims | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 199628475 A
- JP 2010174034 A
- US 4917888 A
- WO 198700056 A
- WO 199955377 A
- JP 2009207490 A


### Non-patent literature cited in the description


- **LIU K.X. et al.** *The American Journal of Physiology,* 1998, vol. 275, E835-E842
- **SAKATA H. et al.** *Cell Growth & Differentiation,* 1996, vol. 7, 1513-1523
- **JAKUBCZAK J.L. et al.** *Molecular and Cellular Biology,* 1998, vol. 18 (3), 1275-1283
- **OTSUKA T. et al.** *Molecular and Cellular Biology,* 2000, vol. 20 (6), 2055-2065
- **DATE K. et al.** *FEBS Letters,* 1997, vol. 420 (1), 1-6
- **ROSS J. et al.** *Gastroenterology,* 2012, vol. 142, 897-906
- **STEFAN N. et al.** *Bioconjugate Chemistry,* 2014, vol. 25, 2144-2156
- **MIYAZAWA K. et al.** *The Journal of Biological Chemistry,* 1996, vol. 271 (7), 3615-3618
- **KINOSAKI M. et al.** *FEBS Letters,* 1998, vol. 434, 165-170
- **ALTSCHUL S. et al.** *Journal of Molecular Biology,* 1990, vol. 215 (3), 403-410
- **ALTSCHUL S. et al.** *Nucleic Acids Research,* 1997, vol. 25 (17), 3389-3402
- **LIETHA D. et al.** *The EMBO Journal,* 2001, vol. 20 (20), 5543-5555
- **JONES D.S. 2ND. et al.** *Proceedings of the National Academy of Sciences of the United States of America,* 2011, vol. 108 (32), 13035-13040
- **MELENHORST W.B. et al.** *American Journal of Physiology,* 2009, vol. 297 (3), 781-790
- **DAVID C.J.H. et al.** *American Journal of Physiology,* 2001, vol. 159 (4), 1383-1393
- **JUNWEI Y. et al.** *The Journal of Clinical Investigation,* 2002, vol. 110, 1525-1538
- **ALDO B. et al.** *Journal of Biological Chemistry,* 2003, vol. 278, 25933-25939
- **BELEN S.J. et al.** *Journal of Biological Chemistry,* 2007, vol. 282, 8325-8331
- **CAESCU C.I. et al.** *Biochemical Journal,* 2009, vol. 424, 79-88
- **MAIKE G. et al.** *PLoS ONE,* 2012, vol. 7, E31756-31771
- **BO C. et al.** *The Journal of Neuroscience,* 2012, vol. 32 (22), 7622-7631
- **INADA et al.** *Journal of Bioact and Compatible Polymers,* 1990, vol. 5, 343
- **DELGADO et al.** *Critical Reviews in Therapeutic Drug Carrier Systems,* 1992, vol. 9, 249
- **KATRE.** *Advanced Drug Delivery Systems,* 1993, vol. 10, 91
- **SANDLER ; KARO.** Polymer Synthesis. Academic Press, vol. 3, 138-161
- **SUNDQVIST T. et al.** *Computers and Biomedical Research,* 1988, vol. 21 (2), 110-116
- Molecular Cloning. Cold Spring harbor Laboratory, 1989
- Bioorganic & Medicinal Chemistry Letters. 2004, vol. 14, 5743-5745
- **RUBIN J.S. et al.** *The Journal of Biological Chemistry,* 2001, vol. 276 (35), 32977-32983
- **LIU Y. et al.** *American Journal of Physiology,* 1999, vol. 277 (4), 624-633

SEQUENCE LISTING

<110>  TORAY INDUSTRIES, INC.

<120>  Hepatocyte growth factor or active fragment thereof modified by polyethylene glycol

<130>  19317WO01

<160>  20

<170>  PatentIn version 3.5

<210>  1
<211>  697
<212>  PRT
<213>  Homo sapiens

<220>
<223>  Human HGF amino acid sequence having no secretion signal sequence

<400>  1

```
Gln Arg Lys Arg Arg Asn Thr Ile His Glu Phe Lys Lys Ser Ala Lys
1               5                   10                  15

Thr Thr Leu Ile Lys Ile Asp Pro Ala Leu Lys Ile Lys Thr Lys Lys
            20                  25                  30

Val Asn Thr Ala Asp Gln Cys Ala Asn Arg Cys Thr Arg Asn Lys Gly
        35                  40                  45

Leu Pro Phe Thr Cys Lys Ala Phe Val Phe Asp Lys Ala Arg Lys Gln
    50                  55                  60

Cys Leu Trp Phe Pro Phe Asn Ser Met Ser Ser Gly Val Lys Lys Glu
65                  70                  75                  80

Phe Gly His Glu Phe Asp Leu Tyr Glu Asn Lys Asp Tyr Ile Arg Asn
                85                  90                  95

Cys Ile Ile Gly Lys Gly Arg Ser Tyr Lys Gly Thr Val Ser Ile Thr
            100                 105                 110

Lys Ser Gly Ile Lys Cys Gln Pro Trp Ser Ser Met Ile Pro His Glu
        115                 120                 125

His Ser Phe Leu Pro Ser Ser Tyr Arg Gly Lys Asp Leu Gln Glu Asn
    130                 135                 140

Tyr Cys Arg Asn Pro Arg Gly Glu Glu Gly Gly Pro Trp Cys Phe Thr
145                 150                 155                 160
```

```
Ser Asn Pro Glu Val Arg Tyr Glu Val Cys Asp Ile Pro Gln Cys Ser
                165                 170                 175

Glu Val Glu Cys Met Thr Cys Asn Gly Glu Ser Tyr Arg Gly Leu Met
            180                 185                 190

Asp His Thr Glu Ser Gly Lys Ile Cys Gln Arg Trp Asp His Gln Thr
        195                 200                 205

Pro His Arg His Lys Phe Leu Pro Glu Arg Tyr Pro Asp Lys Gly Phe
    210                 215                 220

Asp Asp Asn Tyr Cys Arg Asn Pro Asp Gly Gln Pro Arg Pro Trp Cys
225                 230                 235                 240

Tyr Thr Leu Asp Pro His Thr Arg Trp Glu Tyr Cys Ala Ile Lys Thr
                245                 250                 255

Cys Ala Asp Asn Thr Met Asn Asp Thr Asp Val Pro Leu Glu Thr Thr
            260                 265                 270

Glu Cys Ile Gln Gly Gln Gly Glu Gly Tyr Arg Gly Thr Val Asn Thr
        275                 280                 285

Ile Trp Asn Gly Ile Pro Cys Gln Arg Trp Asp Ser Gln Tyr Pro His
    290                 295                 300

Glu His Asp Met Thr Pro Glu Asn Phe Lys Cys Lys Asp Leu Arg Glu
305                 310                 315                 320

Asn Tyr Cys Arg Asn Pro Asp Gly Ser Glu Ser Pro Trp Cys Phe Thr
                325                 330                 335

Thr Asp Pro Asn Ile Arg Val Gly Tyr Cys Ser Gln Ile Pro Asn Cys
            340                 345                 350

Asp Met Ser His Gly Gln Asp Cys Tyr Arg Gly Asn Gly Lys Asn Tyr
        355                 360                 365

Met Gly Asn Leu Ser Gln Thr Arg Ser Gly Leu Thr Cys Ser Met Trp
    370                 375                 380

Asp Lys Asn Met Glu Asp Leu His Arg His Ile Phe Trp Glu Pro Asp
385                 390                 395                 400

Ala Ser Lys Leu Asn Glu Asn Tyr Cys Arg Asn Pro Asp Asp Asp Ala
                405                 410                 415
```

His Gly Pro Trp Cys Tyr Thr Gly Asn Pro Leu Ile Pro Trp Asp Tyr
420 425 430

Cys Pro Ile Ser Arg Cys Glu Gly Asp Thr Thr Pro Thr Ile Val Asn
435 440 445

Leu Asp His Pro Val Ile Ser Cys Ala Lys Thr Lys Gln Leu Arg Val
450 455 460

Val Asn Gly Ile Pro Thr Arg Thr Asn Ile Gly Trp Met Val Ser Leu
465 470 475 480

Arg Tyr Arg Asn Lys His Ile Cys Gly Gly Ser Leu Ile Lys Glu Ser
485 490 495

Trp Val Leu Thr Ala Arg Gln Cys Phe Pro Ser Arg Asp Leu Lys Asp
500 505 510

Tyr Glu Ala Trp Leu Gly Ile His Asp Val His Gly Arg Gly Asp Glu
515 520 525

Lys Cys Lys Gln Val Leu Asn Val Ser Gln Leu Val Tyr Gly Pro Glu
530 535 540

Gly Ser Asp Leu Val Leu Met Lys Leu Ala Arg Pro Ala Val Leu Asp
545 550 555 560

Asp Phe Val Ser Thr Ile Asp Leu Pro Asn Tyr Gly Cys Thr Ile Pro
565 570 575

Glu Lys Thr Ser Cys Ser Val Tyr Gly Trp Gly Tyr Thr Gly Leu Ile
580 585 590

Asn Tyr Asp Gly Leu Leu Arg Val Ala His Leu Tyr Ile Met Gly Asn
595 600 605

Glu Lys Cys Ser Gln His His Arg Gly Lys Val Thr Leu Asn Glu Ser
610 615 620

Glu Ile Cys Ala Gly Ala Glu Lys Ile Gly Ser Gly Pro Cys Glu Gly
625 630 635 640

Asp Tyr Gly Gly Pro Leu Val Cys Glu Gln His Lys Met Arg Met Val
645 650 655

Leu Gly Val Ile Val Pro Gly Arg Gly Cys Ala Ile Pro Asn Arg Pro
660 665 670

Gly Ile Phe Val Arg Val Ala Tyr Tyr Ala Lys Trp Ile His Lys Ile
675 680 685

Ile Leu Thr Tyr Lys Val Pro Gln Ser
690 695

<210> 2
<211> 179
<212> PRT
<213> Homo sapiens

<220>
<223> Human NK1 amino acid sequence having no secretion signal sequence

<400> 2

Gln Arg Lys Arg Arg Asn Thr Ile His Glu Phe Lys Lys Ser Ala Lys
1 5 10 15

Thr Thr Leu Ile Lys Ile Asp Pro Ala Leu Lys Ile Lys Thr Lys Lys
20 25 30

Val Asn Thr Ala Asp Gln Cys Ala Asn Arg Cys Thr Arg Asn Lys Gly
35 40 45

Leu Pro Phe Thr Cys Lys Ala Phe Val Phe Asp Lys Ala Arg Lys Gln
50 55 60

Cys Leu Trp Phe Pro Phe Asn Ser Met Ser Ser Gly Val Lys Lys Glu
65 70 75 80

Phe Gly His Glu Phe Asp Leu Tyr Glu Asn Lys Asp Tyr Ile Arg Asn
85 90 95

Cys Ile Ile Gly Lys Gly Arg Ser Tyr Lys Gly Thr Val Ser Ile Thr
100 105 110

Lys Ser Gly Ile Lys Cys Gln Pro Trp Ser Ser Met Ile Pro His Glu
115 120 125

His Ser Phe Leu Pro Ser Ser Tyr Arg Gly Lys Asp Leu Gln Glu Asn
130 135 140

Tyr Cys Arg Asn Pro Arg Gly Glu Glu Gly Gly Pro Trp Cys Phe Thr
145 150 155 160

Ser Asn Pro Glu Val Arg Tyr Glu Val Cys Asp Ile Pro Gln Cys Ser
165 170 175

Glu Val Glu

<210> 3
<211> 31
<212> PRT
<213> Homo sapiens

<220>
<223> Human HGF-derived secretion signal sequence

<400> 3

```
Met Trp Val Thr Lys Leu Leu Pro Ala Leu Leu Leu Gln His Val Leu
1               5                   10                  15


Leu His Leu Leu Leu Leu Pro Ile Ala Ile Pro Tyr Ala Glu Gly
            20                  25                  30
```

<210> 4
<211> 7
<212> PRT
<213> Artificial

<220>
<223> Amino acid sequence of cysteine residue and\201@6xHis tag, added to the amino terminus of human NK1 protein

<400> 4

```
Cys His His His His His His
1               5
```

<210> 5
<211> 7
<212> PRT
<213> Artificial

<220>
<223> Amino acid sequence of 6xHis tag and cysteine residue, added to the carboxyl terminus of human NK1 protein

<400> 5

```
His His His His His His Cys
1               5
```

<210> 6
<211> 2576
<212> DNA
<213> Homo sapiens

<220>
<221> CDS
<222> (102)..(2288)

<400> 6

```
gggctcagag ccgactggct cttttaggca ctgactccga acaggattct ttcacccagg      60

catctcctcc agagggatcc gccagcccgt ccagcagcac c atg tgg gtg acc aaa     116
                                              Met Trp Val Thr Lys
                                              1               5

ctc ctg cca gcc ctg ctg ctg cag cat gtc ctc ctg cat ctc ctc ctg       164
Leu Leu Pro Ala Leu Leu Leu Gln His Val Leu Leu His Leu Leu Leu
                10                  15                  20

ctc ccc atc gcc atc ccc tat gca gag gga caa agg aaa aga aga aat       212
Leu Pro Ile Ala Ile Pro Tyr Ala Glu Gly Gln Arg Lys Arg Arg Asn
            25                  30                  35

aca att cat gaa ttc aaa aaa tca gca aag act acc cta atc aaa ata       260
Thr Ile His Glu Phe Lys Lys Ser Ala Lys Thr Thr Leu Ile Lys Ile
        40                  45                  50

gat cca gca ctg aag ata aaa acc aaa aaa gtg aat act gca gac caa       308
Asp Pro Ala Leu Lys Ile Lys Thr Lys Lys Val Asn Thr Ala Asp Gln
    55                  60                  65

tgt gct aat aga tgt act agg aat aaa gga ctt cca ttc act tgc aag       356
Cys Ala Asn Arg Cys Thr Arg Asn Lys Gly Leu Pro Phe Thr Cys Lys
70                  75                  80                  85

gct ttt gtt ttt gat aaa gca aga aaa caa tgc ctc tgg ttc ccc ttc       404
Ala Phe Val Phe Asp Lys Ala Arg Lys Gln Cys Leu Trp Phe Pro Phe
                90                  95                  100

aat agc atg tca agt gga gtg aaa aaa gaa ttt ggc cat gaa ttt gac       452
Asn Ser Met Ser Ser Gly Val Lys Lys Glu Phe Gly His Glu Phe Asp
            105                 110                 115

ctc tat gaa aac aaa gac tac att aga aac tgc atc att ggt aaa gga       500
Leu Tyr Glu Asn Lys Asp Tyr Ile Arg Asn Cys Ile Ile Gly Lys Gly
        120                 125                 130

cgc agc tac aag gga aca gta tct atc act aag agt ggc atc aaa tgt       548
Arg Ser Tyr Lys Gly Thr Val Ser Ile Thr Lys Ser Gly Ile Lys Cys
    135                 140                 145

cag ccc tgg agt tcc atg ata cca cac gaa cac agc ttt ttg cct tcg       596
Gln Pro Trp Ser Ser Met Ile Pro His Glu His Ser Phe Leu Pro Ser
150                 155                 160                 165

agc tat cgg ggt aaa gac cta cag gaa aac tac tgt cga aat cct cga       644
Ser Tyr Arg Gly Lys Asp Leu Gln Glu Asn Tyr Cys Arg Asn Pro Arg
                170                 175                 180

ggg gaa gaa ggg gga ccc tgg tgt ttc aca agc aat cca gag gta cgc       692
Gly Glu Glu Gly Gly Pro Trp Cys Phe Thr Ser Asn Pro Glu Val Arg
            185                 190                 195

tac gaa gtc tgt gac att cct cag tgt tca gaa gtt gaa tgc atg acc       740
Tyr Glu Val Cys Asp Ile Pro Gln Cys Ser Glu Val Glu Cys Met Thr
        200                 205                 210

tgc aat ggg gag agt tat cga ggt ctc atg gat cat aca gaa tca ggc       788
Cys Asn Gly Glu Ser Tyr Arg Gly Leu Met Asp His Thr Glu Ser Gly
    215                 220                 225
```

```
aag att tgt cag cgc tgg gat cat cag aca cca cac cgg cac aaa ttc        836
Lys Ile Cys Gln Arg Trp Asp His Gln Thr Pro His Arg His Lys Phe
230                 235                 240                 245

ttg cct gaa aga tat ccc gac aag ggc ttt gat gat aat tat tgc cgc        884
Leu Pro Glu Arg Tyr Pro Asp Lys Gly Phe Asp Asp Asn Tyr Cys Arg
                250                 255                 260

aat ccc gat ggc cag ccg agg cca tgg tgc tat act ctt gac cct cac        932
Asn Pro Asp Gly Gln Pro Arg Pro Trp Cys Tyr Thr Leu Asp Pro His
            265                 270                 275

acc cgc tgg gag tac tgt gca att aaa aca tgc gct gac aat act atg        980
Thr Arg Trp Glu Tyr Cys Ala Ile Lys Thr Cys Ala Asp Asn Thr Met
        280                 285                 290

aat gac act gat gtt cct ttg gaa aca act gaa tgc atc caa ggt caa       1028
Asn Asp Thr Asp Val Pro Leu Glu Thr Thr Glu Cys Ile Gln Gly Gln
    295                 300                 305

gga gaa ggc tac agg ggc act gtc aat acc att tgg aat gga att cca       1076
Gly Glu Gly Tyr Arg Gly Thr Val Asn Thr Ile Trp Asn Gly Ile Pro
310                 315                 320                 325

tgt cag cgt tgg gat tct cag tat cct cac gag cat gac atg act cct       1124
Cys Gln Arg Trp Asp Ser Gln Tyr Pro His Glu His Asp Met Thr Pro
                330                 335                 340

gaa aat ttc aag tgc aag gac cta cga gaa aat tac tgc cga aat cca       1172
Glu Asn Phe Lys Cys Lys Asp Leu Arg Glu Asn Tyr Cys Arg Asn Pro
            345                 350                 355

gat ggg tct gaa tca ccc tgg tgt ttt acc act gat cca aac atc cga       1220
Asp Gly Ser Glu Ser Pro Trp Cys Phe Thr Thr Asp Pro Asn Ile Arg
        360                 365                 370

gtt ggc tac tgc tcc caa att cca aac tgt gat atg tca cat gga caa       1268
Val Gly Tyr Cys Ser Gln Ile Pro Asn Cys Asp Met Ser His Gly Gln
    375                 380                 385

gat tgt tat cgt ggg aat ggc aaa aat tat atg ggc aac tta tcc caa       1316
Asp Cys Tyr Arg Gly Asn Gly Lys Asn Tyr Met Gly Asn Leu Ser Gln
390                 395                 400                 405

aca aga tct gga cta aca tgt tca atg tgg gac aag aac atg gaa gac       1364
Thr Arg Ser Gly Leu Thr Cys Ser Met Trp Asp Lys Asn Met Glu Asp
                410                 415                 420

tta cat cgt cat atc ttc tgg gaa cca gat gca agt aag ctg aat gag       1412
Leu His Arg His Ile Phe Trp Glu Pro Asp Ala Ser Lys Leu Asn Glu
            425                 430                 435

aat tac tgc cga aat cca gat gat gat gct cat gga ccc tgg tgc tac       1460
Asn Tyr Cys Arg Asn Pro Asp Asp Asp Ala His Gly Pro Trp Cys Tyr
        440                 445                 450

acg gga aat cca ctc att cct tgg gat tat tgc cct att tct cgt tgt       1508
Thr Gly Asn Pro Leu Ile Pro Trp Asp Tyr Cys Pro Ile Ser Arg Cys
    455                 460                 465

gaa ggt gat acc aca cct aca ata gtc aat tta gac cat ccc gta ata       1556
Glu Gly Asp Thr Thr Pro Thr Ile Val Asn Leu Asp His Pro Val Ile
470                 475                 480                 485
```

```
tct tgt gcc aaa acg aaa caa ttg cga gtt gta aat ggg att cca aca        1604
Ser Cys Ala Lys Thr Lys Gln Leu Arg Val Val Asn Gly Ile Pro Thr
                490                 495                 500

cga aca aac ata gga tgg atg gtt agt ttg aga tac aga aat aaa cat        1652
Arg Thr Asn Ile Gly Trp Met Val Ser Leu Arg Tyr Arg Asn Lys His
            505                 510                 515

atc tgc gga gga tca ttg ata aag gag agt tgg gtt ctt act gca cga        1700
Ile Cys Gly Gly Ser Leu Ile Lys Glu Ser Trp Val Leu Thr Ala Arg
        520                 525                 530

cag tgt ttc cct tct cga gac ttg aaa gat tat gaa gct tgg ctt gga        1748
Gln Cys Phe Pro Ser Arg Asp Leu Lys Asp Tyr Glu Ala Trp Leu Gly
    535                 540                 545

att cat gat gtc cac gga aga gga gat gag aaa tgc aaa cag gtt ctc        1796
Ile His Asp Val His Gly Arg Gly Asp Glu Lys Cys Lys Gln Val Leu
550                 555                 560                 565

aat gtt tcc cag ctg gta tat ggc cct gaa gga tca gat ctg gtt tta        1844
Asn Val Ser Gln Leu Val Tyr Gly Pro Glu Gly Ser Asp Leu Val Leu
                570                 575                 580

atg aag ctt gcc agg cct gct gtc ctg gat gat ttt gtt agt acg att        1892
Met Lys Leu Ala Arg Pro Ala Val Leu Asp Asp Phe Val Ser Thr Ile
            585                 590                 595

gat tta cct aat tat gga tgc aca att cct gaa aag acc agt tgc agt        1940
Asp Leu Pro Asn Tyr Gly Cys Thr Ile Pro Glu Lys Thr Ser Cys Ser
        600                 605                 610

gtt tat ggc tgg ggc tac act gga ttg atc aac tat gat ggc cta tta        1988
Val Tyr Gly Trp Gly Tyr Thr Gly Leu Ile Asn Tyr Asp Gly Leu Leu
    615                 620                 625

cga gtg gca cat ctc tat ata atg gga aat gag aaa tgc agc cag cat        2036
Arg Val Ala His Leu Tyr Ile Met Gly Asn Glu Lys Cys Ser Gln His
630                 635                 640                 645

cat cga ggg aag gtg act ctg aat gag tct gaa ata tgt gct ggg gct        2084
His Arg Gly Lys Val Thr Leu Asn Glu Ser Glu Ile Cys Ala Gly Ala
                650                 655                 660

gaa aag att gga tca gga cca tgt gag ggg gat tat ggt ggc cca ctt        2132
Glu Lys Ile Gly Ser Gly Pro Cys Glu Gly Asp Tyr Gly Gly Pro Leu
            665                 670                 675

gtt tgt gag caa cat aaa atg aga atg gtt ctt ggt gtc att gtt cct        2180
Val Cys Glu Gln His Lys Met Arg Met Val Leu Gly Val Ile Val Pro
        680                 685                 690

ggt cgt gga tgt gcc att cca aat cgt cct ggt att ttt gtc cga gta        2228
Gly Arg Gly Cys Ala Ile Pro Asn Arg Pro Gly Ile Phe Val Arg Val
    695                 700                 705

gca tat tat gca aaa tgg ata cac aaa att att tta aca tat aag gta        2276
Ala Tyr Tyr Ala Lys Trp Ile His Lys Ile Ile Leu Thr Tyr Lys Val
710                 715                 720                 725

cca cag tca tag ctgaagtaag tgtgtctgaa gcacccacca atacaactgt            2328
Pro Gln Ser
```

```
cttttacatg aagatttcag agaatgtgga atttaaaatg tcacttacaa caatcctaag      2388

acaactactg gagagtcatg tttgttgaaa ttctcattaa tgtttatggg tgttttctgt      2448

tgttttgttt gtcagtgtta ttttgtcaat gttgaagtga attaaggtac atgcaagtgt      2508

aataacatat ctcctgaaga tacttgaatg gattaaaaaa acacacaggt atatttgctg      2568

gatgataa                                                               2576


<210>  7
<211>  728
<212>  PRT
<213>  Homo sapiens

<400>  7

Met Trp Val Thr Lys Leu Leu Pro Ala Leu Leu Leu Gln His Val Leu
1               5                   10                  15


Leu His Leu Leu Leu Leu Pro Ile Ala Ile Pro Tyr Ala Glu Gly Gln
            20                  25                  30


Arg Lys Arg Arg Asn Thr Ile His Glu Phe Lys Lys Ser Ala Lys Thr
        35                  40                  45


Thr Leu Ile Lys Ile Asp Pro Ala Leu Lys Ile Lys Thr Lys Lys Val
    50                  55                  60


Asn Thr Ala Asp Gln Cys Ala Asn Arg Cys Thr Arg Asn Lys Gly Leu
65                  70                  75                  80


Pro Phe Thr Cys Lys Ala Phe Val Phe Asp Lys Ala Arg Lys Gln Cys
                85                  90                  95


Leu Trp Phe Pro Phe Asn Ser Met Ser Ser Gly Val Lys Lys Glu Phe
            100                 105                 110


Gly His Glu Phe Asp Leu Tyr Glu Asn Lys Asp Tyr Ile Arg Asn Cys
        115                 120                 125


Ile Ile Gly Lys Gly Arg Ser Tyr Lys Gly Thr Val Ser Ile Thr Lys
    130                 135                 140


Ser Gly Ile Lys Cys Gln Pro Trp Ser Ser Met Ile Pro His Glu His
145                 150                 155                 160


Ser Phe Leu Pro Ser Ser Tyr Arg Gly Lys Asp Leu Gln Glu Asn Tyr
                165                 170                 175
```

Cys Arg Asn Pro Arg Gly Glu Glu Gly Gly Pro Trp Cys Phe Thr Ser
180 185 190

Asn Pro Glu Val Arg Tyr Glu Val Cys Asp Ile Pro Gln Cys Ser Glu
195 200 205

Val Glu Cys Met Thr Cys Asn Gly Glu Ser Tyr Arg Gly Leu Met Asp
210 215 220

His Thr Glu Ser Gly Lys Ile Cys Gln Arg Trp Asp His Gln Thr Pro
225 230 235 240

His Arg His Lys Phe Leu Pro Glu Arg Tyr Pro Asp Lys Gly Phe Asp
245 250 255

Asp Asn Tyr Cys Arg Asn Pro Asp Gly Gln Pro Arg Pro Trp Cys Tyr
260 265 270

Thr Leu Asp Pro His Thr Arg Trp Glu Tyr Cys Ala Ile Lys Thr Cys
275 280 285

Ala Asp Asn Thr Met Asn Asp Thr Asp Val Pro Leu Glu Thr Thr Glu
290 295 300

Cys Ile Gln Gly Gln Gly Glu Gly Tyr Arg Gly Thr Val Asn Thr Ile
305 310 315 320

Trp Asn Gly Ile Pro Cys Gln Arg Trp Asp Ser Gln Tyr Pro His Glu
325 330 335

His Asp Met Thr Pro Glu Asn Phe Lys Cys Lys Asp Leu Arg Glu Asn
340 345 350

Tyr Cys Arg Asn Pro Asp Gly Ser Glu Ser Pro Trp Cys Phe Thr Thr
355 360 365

Asp Pro Asn Ile Arg Val Gly Tyr Cys Ser Gln Ile Pro Asn Cys Asp
370 375 380

Met Ser His Gly Gln Asp Cys Tyr Arg Gly Asn Gly Lys Asn Tyr Met
385 390 395 400

Gly Asn Leu Ser Gln Thr Arg Ser Gly Leu Thr Cys Ser Met Trp Asp
405 410 415

Lys Asn Met Glu Asp Leu His Arg His Ile Phe Trp Glu Pro Asp Ala
420 425 430

Ser Lys Leu Asn Glu Asn Tyr Cys Arg Asn Pro Asp Asp Asp Ala His
435 440 445

Gly Pro Trp Cys Tyr Thr Gly Asn Pro Leu Ile Pro Trp Asp Tyr Cys
450 455 460

Pro Ile Ser Arg Cys Glu Gly Asp Thr Thr Pro Thr Ile Val Asn Leu
465 470 475 480

Asp His Pro Val Ile Ser Cys Ala Lys Thr Lys Gln Leu Arg Val Val
485 490 495

Asn Gly Ile Pro Thr Arg Thr Asn Ile Gly Trp Met Val Ser Leu Arg
500 505 510

Tyr Arg Asn Lys His Ile Cys Gly Gly Ser Leu Ile Lys Glu Ser Trp
515 520 525

Val Leu Thr Ala Arg Gln Cys Phe Pro Ser Arg Asp Leu Lys Asp Tyr
530 535 540

Glu Ala Trp Leu Gly Ile His Asp Val His Gly Arg Gly Asp Glu Lys
545 550 555 560

Cys Lys Gln Val Leu Asn Val Ser Gln Leu Val Tyr Gly Pro Glu Gly
565 570 575

Ser Asp Leu Val Leu Met Lys Leu Ala Arg Pro Ala Val Leu Asp Asp
580 585 590

Phe Val Ser Thr Ile Asp Leu Pro Asn Tyr Gly Cys Thr Ile Pro Glu
595 600 605

Lys Thr Ser Cys Ser Val Tyr Gly Trp Gly Tyr Thr Gly Leu Ile Asn
610 615 620

Tyr Asp Gly Leu Leu Arg Val Ala His Leu Tyr Ile Met Gly Asn Glu
625 630 635 640

Lys Cys Ser Gln His His Arg Gly Lys Val Thr Leu Asn Glu Ser Glu
645 650 655

Ile Cys Ala Gly Ala Glu Lys Ile Gly Ser Gly Pro Cys Glu Gly Asp
660 665 670

Tyr Gly Gly Pro Leu Val Cys Glu Gln His Lys Met Arg Met Val Leu

675 680 685

Gly Val Ile Val Pro Gly Arg Gly Cys Ala Ile Pro Asn Arg Pro Gly
690 695 700

Ile Phe Val Arg Val Ala Tyr Tyr Ala Lys Trp Ile His Lys Ile Ile
705 710 715 720

Leu Thr Tyr Lys Val Pro Gln Ser

<210> 8
<211> 12
<212> PRT
<213> Homo sapiens

<400> 8

Ser Pro Leu Ala Gln Ala Val Arg Ser Ser Ser Arg
1 5 10

<210> 9
<211> 9
<212> PRT
<213> Artificial

<220>
<223> Artificial ADAM-17 cleavable sequence

<400> 9

Pro Arg Ala Ala Ala Val Lys Ser Pro
1 5

<210> 10
<211> 9
<212> PRT
<213> Homo sapiens

<400> 10

Gln Glu Thr Asn Arg Ser Phe Ser Lys
1 5

<210> 11
<211> 9
<212> PRT
<213> Homo sapiens

<400> 11

Pro Arg Val Ala Gln Val Ser Ile Thr
1 5

<210> 12
<211> 9

<212> PRT
<213> Homo sapiens

<400> 12

Pro Val Ala Ala Ala Val Val Ser His
1 5

<210> 13
<211> 9
<212> PRT
<213> Homo sapiens

<400> 13

Ala Asp Leu Leu Ala Val Val Ala Ala
1 5

<210> 14
<211> 9
<212> PRT
<213> Artificial

<220>
<223> Variant sequence 1 of SEQ No.9

<400> 14

Pro Arg Ala Ala Ala Leu Lys Ser Pro
1 5

<210> 15
<211> 9
<212> PRT
<213> Artificial

<220>
<223> Variant sequence 2 of SEQ No.9

<400> 15

Pro Arg Ala Ala Ala Tyr Lys Ser Pro
1 5

<210> 16
<211> 9
<212> PRT
<213> Artificial

<220>
<223> Variant sequence 3 of SEQ No.9

<400> 16

Pro Arg Ala Ala Ala Ala Lys Ser Pro
1 5

<210> 17

```
<211>  11
<212>  PRT
<213>  Artificial

<220>
<223>  Artificial Thrombin cleavable  sequence

<400>  17

Thr Phe Leu Thr Pro Arg Gly Val Arg Leu Gly
1               5                   10


<210>  18
<211>  9
<212>  PRT
<213>  Artificial

<220>
<223>  Artificial Thrombin cleavable  sequence

<400>  18

Thr Phe Pro Pro Arg Ser Phe Leu Gly
1               5


<210>  19
<211>  8
<212>  PRT
<213>  Homo sapiens

<220>
<223>  Thrombin cleavable sequence derived from Human Prothrombin

<400>  19

Phe Asn Pro Arg Thr Phe Gly Ser
1               5


<210>  20
<211>  8
<212>  PRT
<213>  Homo sapiens

<220>
<223>  Thrombin cleavable sequence derived from Human HGFA

<400>  20

Leu Arg Pro Arg Ile Ile Gly Gly
1               5
```